(19) Europäisches Patentamt
European Patent Office
Office européen des brevets

(11) EP 2 906 956 B1

(12) EUROPEAN PATENT SPECIFICATION

(45) Date of publication and mention of the grant of the patent:
**25.11.2020 Bulletin 2020/48**

(21) Application number: **13844778.4**

(22) Date of filing: **09.10.2013**

(51) Int Cl.:
*G01R 33/465* (2006.01)    *G01N 33/68* (2006.01)
*G01N 33/50* (2006.01)    *G01N 21/31* (2006.01)
*G01R 33/46* (2006.01)

(86) International application number:
**PCT/US2013/064142**

(87) International publication number:
**WO 2014/059025 (17.04.2014 Gazette 2014/16)**

(54) **NMR QUANTIFICATION OF BRANCHED CHAIN AMINO ACIDS**

NMR-QUANTIFIZIERUNG VON VERZWEIGTKETTIGEN AMINOSÄUREN

QUANTIFICATION PAR NMR D'ACIDES AMINÉS À CHAÎNE RAMIFIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 09.10.2012 US 201261711471 P
13.03.2013 US 201313801604
14.03.2013 US 201313830784
14.03.2013 US 201313830199
16.05.2013 PCT/US2013/041274
30.05.2013 PCT/US2013/043343
05.06.2013 US 201361831353 P
07.06.2013 PCT/US2013/044679

(43) Date of publication of application:
**19.08.2015 Bulletin 2015/34**

(60) Divisional application:
**20199972.9**

(73) Proprietor: **Liposcience, Inc.**
**Raleigh, NC 27616 (US)**

(72) Inventors:
• **O'CONNELL, Thomas M.**
**Chapel Hill**
**North Carolina 27517 (US)**
• **MERCIER, Kelly A.**
**Raleigh**
**North Carolina 27616 (US)**
• **SHALAUROVA, Irina Y.**
**Cary**
**North Carolina 27519 (US)**
• **OTVOS, James D.**
**Apex**
**North Carolina 27502 (US)**

(74) Representative: **Yeadon IP Limited**
**Nexus**
**Discovery Way**
**Leeds LS2 3AA (GB)**

(56) References cited:
EP-B1- 0 264 815      WO-A1-2009/064843
WO-A1-2010/128054    WO-A1-2011/011781
US-A1- 2011 189 780    US-A1- 2011 275 985

• Nikolaos Psychogios ET AL: "The Human Serum Metabolome", PLoS ONE, 16 February 2011 (2011-02-16), pages 1-23, XP055274573, Internet Retrieved from the Internet: URL:http://journals.plos.org/plosone/artic le?id=10.1371/journal.pone.0016957 [retrieved on 2016-05-23]
• WISHART D S ET AL: "The human cerebrospinal fluid metabolome", JOURNAL OF CHROMATOGRAPHY B: BIOMEDICAL SCIENCES & APPLICATIONS, ELSEVIER, AMSTERDAM, NL, vol. 871, no. 2, 15 August 2008 (2008-08-15), pages 164-173, XP023782691, ISSN: 1570-0232, DOI: 10.1016/J.JCHROMB.2008.05.001 [retrieved on 2008-05-08]

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- WEI ET AL: "Toxicological effects of cinnabar in rats by NMR-based metabolic profiling of urine and serum", TOXICOLOGY AND APPLIED PHARMACOLOGY, ACADEMIC PRESS, AMSTERDAM, NL, vol. 227, no. 3, 31 December 2007 (2007-12-31), pages 417-429, XP022510442, ISSN: 0041-008X, DOI: 10.1016/J.TAAP.2007.11.015
- HILTUNEN Y ET AL: "A LINESHAPE FITTING MODEL FOR 1H NMR SPECTRA OF HUMAN BLOOD PLASMA", MAGNETIC RESONANCE IN MEDICINE, JOHN WILEY & SONS, INC, US, vol. 21, no. 2, 1 October 1991 (1991-10-01), pages 222-232, XP000235327, ISSN: 0740-3194
- GAO H ET AL: "Metabonomic profiling of renal cell carcinoma: High-resolution proton nuclear magnetic resonance spectroscopy of human serum with multivariate data analysis", ANALYTICA CHIMICA ACTA, ELSEVIER, AMSTERDAM, NL, vol. 624, no. 2, 29 August 2008 (2008-08-29), pages 269-277, XP023785540, ISSN: 0003-2670, DOI: 10.1016/J.ACA.2008.06.051 [retrieved on 2008-07-06]
- GUIDO F. PAULI ET AL: "Quantitative 1 H NMR. Development and Potential of an Analytical Method: An Update", JOURNAL OF NATURAL PRODUCTS., vol. 75, no. 4, 27 April 2012 (2012-04-27) , pages 834-851, XP055245546, US ISSN: 0163-3864, DOI: 10.1021/np200993k

**Description**

**Field of the Invention**

The present invention relates generally to the quantification of branched chain amino acids using NMR. The invention may be particularly suitable for NMR analysis of human blood plasma or serum.

**Background**

Branched chain amino acids (BCAAs), which include valine, isoleucine and leucine, may have associations with various diseases, such as metabolic diseases. They may also be used in therapeutic interventions and as dietary supplements. The article Psychogios N. et al., "The Human Serum Metabolome", PLOS ONE, vol. 6, no. 2, February 2011, discloses a method of determining concentrations of BCAAs in ultrafiltered serum samples, wherein an NMR spectrum including peaks associated with a plurality of BCAAs is deconvolved using defined curve fitting functions. The BCAAs comprise valine, isoleucine and leucine.

**Summary**

The invention concerns a method of determining concentrations of BCAAs, comprising placing at least one unfiltered *in vitro* serum or plasma sample in an NMR spectrometer having a static magnetic field Bo; electronically obtaining an NMR spectrum of the sample that includes peaks associated with a plurality of BCAAs; deconvolving the NMR spectrum using defined curve fitting functions, wherein the deconvolving is carried out using a baseline that is fit using protein and lipoprotein components; computing unitless intensity signals from the deconvolved spectrum associated with respective BCAAs; then electronically calculating concentrations of the plurality of BCAAs using respective correlations of values of the computed intensity signals with known concentrations of corresponding BCAAs, wherein the BCAAs comprise valine, isoleucine and leucine.

The electronically calculating concentrations can be carried out for each of the plurality of BCAAs using a respective defined linear equation associated with concentrations over at least a normal biological range.

The linear equation can optionally also include outliers of high and/or low values.

The method can include for each NMR spectrum of each respective sample before the deconvolving, electronically evaluating at least one reference peak in the NMR spectrum to assess magnetic field inhomogeneity and/or to select an appropriate fitting function or linewidth thereof. The linewidth and/or lineshape of the at least one reference peak can be associated with magnetic field Bo inhomogeneity and/or homogeneity.

The method can include applying scalar coupling information to BCAA peaks in the obtained NMR spectrum to thereby facilitate fitting during the deconvolving.

The scalar coupling information can identify multiple peaks which are separated by a consistent distance as measured in Hertz, irrespective of magnetic field strength, and the deconvolving can include fitting functions that can be constrained to a separation distance at or near the distance to thereby constrain the fitting.

The method can include for each sample, before the deconvolving, electronically determining a location, lineshape and/or linewidth of at least one endogenous or exogenous reference peak(s) present in respective samples that has shape characteristics dependent on homogeneity of Bo.

The method can include computationally determining lineshape and/or linewidth characteristics of each NMR spectrum for respective samples before the deconvolving step using at least one reference peak to account for differences in magnetic field homogeneity characteristics associated with a respective individual NMR spectrum, then selecting a defined deconvolution model with fitting functions having a linewidth and/or lineshape corresponding to the at least one reference peak such that different samples can be evaluated differently using (a) different fitting functions and/or (b) different linewidths.

In some embodiments, the electronically calculating the concentrations can include applying a scalar concentration value defined as a linear function of concentrations versus unitless intensity signal correlations for each BCAA.

In some embodiments, the plurality of BCAAs can include valine, isoleucine and leucine, and the deconvolving can be carried out using a baseline that is fit using protein and lipoprotein components.

The method can include, before the deconvolving, electronically evaluating at least one pH chemical-shift invariant reference peak in the obtained NMR spectrum associated with an endogenous or exogenous analyte or component of the sample, then determining a location of BCAA peaks in the NMR spectrum.

In some embodiments, the electronically obtaining can be carried out using between about 10 seconds to about 5 minutes of NMR acquisition time (AT) for a single NMR spectrum per sample.

In some embodiments, the electronically obtaining can be carried out using between about 16-96 scans for a respective sample NMR spectrum, and the electronically calculating the BCAA concentrations can be carried out using a single NMR spectrum per sample.

The method can include transmitting a pulse sequence that attenuates signal from lipoproteins and proteins in the sample to a greater degree than signal from small molecule BCAAs.

In some embodiments, the pulse sequence can include a Carr-Purcel-Meiboom-Gill (CPMG) pulse sequence with a defined delay that attenuates the lipoprotein and protein signals.

The CPMG pulse sequence can have a delay of between about 60 ms to about 400 ms. In some embodiments, the CPMG pulse sequence can have a delay of about 100 ms.

According to some embodiments, the CPMG pulse sequence can have a delay of about 200 ms, and the method can include electronically correcting the calculated concentrations for quantification errors associated with attenuation of signal intensity of the BCAAs associated with the CPMG pulse sequence.

In some embodiments, the CPMG pulse sequence can have a delay of about 300 ms, and the method can include electronically correcting the calculated concentrations for quantification errors associated with attenuation of signal intensity of the BCAAs associated with the CPMG pulse sequence.

In further embodiments, the CPMG pulse sequence can have a delay of about 400 ms, and the method can include electronically correcting the calculated concentrations for quantification errors associated with attenuation of signal intensity of the BCAAs associated with the CPMG pulse sequence.

In certain embodiments, the method can include transmitting a first lipoprotein pulse sequence and obtaining a first NMR spectrum associated with lipoproteins and proteins in the sample while the sample is in the NMR spectrometer and transmitting a second pulse sequence to obtain the NMR spectrum associated with the BCAAs that is configured to attenuate protein and lipoprotein signals before or after the first pulse sequence to obtain the NMR spectrum of the BCAAs. Both pulse sequences and subsequent NMR spectra can be obtained within about 2 minutes to about 6 minutes of NMR signal acquisition time per sample.

In some embodiments, the method can include flowing respective samples into the NMR spectrometer before the obtaining step. The obtaining can be carried out by serially obtaining respective NMR spectrums at a rate that is between about 50 and 250 unfiltered samples per 8 hours.

In some embodiments, the plurality of BCAAs includes valine, isoleucine and leucine, and valine and leucine can be quantified using less scans associated with the obtained NMR spectrum than isoleucine.

In some embodiments, the NMR spectrum used to calculate valine and leucine can be generated using between 16-96 scans. The NMR spectrum used to calculate isoleucine can be generated using 96 scans.

In some embodiments, the electronically calculating can be carried out using a respective defined linear concentration equation for each BCAA to provide a clinically relevant concentration measurement of at least normal biological concentration ranges.

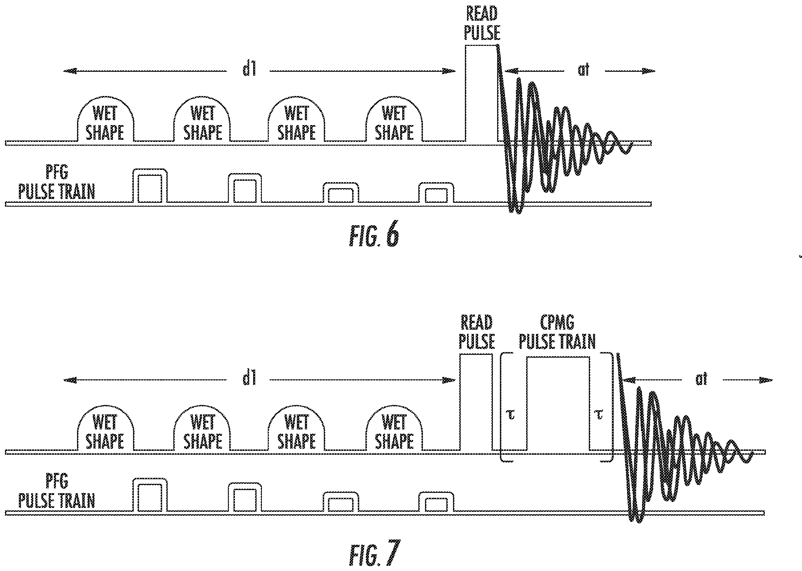

In some embodiments, the first pulse sequence can include the pulse sequence shown in Figure 6.

The method can include, in some embodiments, electronically calculating a ratio or weighted index that is predictive of a clinical outcome or disease risk or identifies an efficacy of a drug or drug candidate. The ratio or weighted index can have at least one of the following: (a) a numerator comprising at least one of the unitless signals or calculated concentrations of one or more of the BCAAs; (b) a denominator comprising at least one of the unitless signals or calculated concentrations of one or more of the BCAAs; or (c) a numerator and a denominator that includes at least one of the unitless signals or calculated concentrations of one or more of the BCAAs.

In some embodiments, the method can include electronically calculating a ratio and/or weighted index that is predictive of a clinical outcome or disease risk or identifies an efficacy of a drug or drug candidate. The ratio or weighted index includes in the denominator or numerator or in both the denominator and numerator at least one of the unitless signals or calculated concentrations of one or more of the BCAAs.

The method can include, in some embodiments, electronically calculating a ratio and/or weighted index that is predictive of a clinical outcome or disease risk or identifies an efficacy of a drug or drug candidate, wherein the ratio and/or weighted index includes in the denominator or numerator or in both the denominator and numerator at least one of the unitless signals or calculated concentrations of one or more of the BCAAs and a unitless signal or concentration measurement of an amino acid.

Certain embodiments of the present invention are directed to a circuit configured to provide measurements of BCAAs. The circuit includes at least one processor configured to carry out the steps of any of the methods of the present invention.

A further aspect of the present invention is directed to a computer program product for evaluating *in vitro* patient serum or plasma biosamples. The computer program product includes: a non-transitory computer readable storage medium having computer readable program code embodied in the medium. The computer-readable program code includes computer readable program code configured to deconvolve a NMR spectrum with Branched Chain Amino Acid (BCAA) peaks of respective unfiltered patient samples using defined fitting functions; wherein the deconvolving is carried out using a baseline that is fit using protein and lipoprotein components; computer readable program code that computes unitless intensity signals from the deconvolved spectrum associated with respective BCAAs; and computer readable program code configured to provide concentrations of the BCAAs for the unitless intensity signals, using respective correlations of values of the computed intensity signals with known concentrations of corresponding BCAAs, wherein the BCAAs comprise valine, isoleucine and leucine.

The invention also concerns a system. The system includes an NMR spectrometer for acquiring at least one NMR spectrum of respective *in vitro* serum or plasma biosamples, the NMR spectrometer having a static magnetic field Bo; and at least one processor in communication with the NMR spectrometer. The at least one processor is configured to, for a respective biosample, using the acquired at least one NMR spectrum: deconvolve the NMR spectrum using defined fitting functions, wherein the deconvolving is carried out using a baseline fit using protein and lipoprotein components; compute unitless intensity signals from the deconvolved spectrum associated with respective BCAAs; then calculate concentrations of the plurality of BCAAs using respective correlations of values of the computed intensity signals with known concentrations of corresponding BCAAs, wherein the BCAAs comprise valine, isoleucine and leucine.

In some embodiments, the at least one processor can be configured to computationally determine lineshape and/or linewidth characteristics of each NMR spectrum for respective samples before the deconvolving step using at least one reference peak to account for differences in magnetic field homogeneity characteristics associated with a respective individual NMR spectrum, then select a defined deconvolution model with fitting functions having a linewidth and/or lineshape corresponding to the at least one reference peak such that different samples can be evaluated differently using (a) different fitting functions and/or (b) different linewidths.

In some embodiments, the at least one processor can be configured to, before deconvolving respective NMR spectrum for quantifying BCAA signals, evaluate at least one pH chemical-shift invariant reference peak in respective obtained NMR spectrums associated with an endogenous or exogenous analyte or component of the sample, then determine a location of BCAA peaks in the NMR spectrum.

In some embodiments, the at least one processor can be configured to calculate a ratio and/or weighted index that is predictive of a clinical outcome or disease risk or identifies an efficacy of a drug or drug candidate. The ratio and/or weighted index can include in the denominator or numerator or in both the denominator and numerator at least one of the unitless signals or calculated concentrations of one or more of the BCAAs.

The at least one processor can, in some embodiments, calculate respective concentrations by applying a scalar concentration value defined as a linear function of concentrations versus unitless intensity signal correlations for each BCAA.

In some embodiments, the BCAAs can include valine, isoleucine and leucine, and the deconvolving can be carried out using a baseline that is fit using protein and lipoprotein components.

In some embodiments, the system can be configured to transmit a first lipoprotein pulse sequence to obtain a first NMR spectrum associated with lipoproteins and proteins in the sample while the sample is in the NMR spectrometer and transmit a second pulse sequence to obtain the NMR spectrum associated with the BCAAs that is configured to attenuate protein and lipoprotein signals before or after the first pulse sequence to obtain the NMR spectrum of the BCAAs. Both pulse sequences and subsequent respective NMR spectrums can be obtained within about 2 minutes to about 6 minutes of NMR signal acquisition time per sample.

In some embodiments, the at least one processor can be configured to carry out any of the methods of the present invention.

Further features, advantages and details of the present invention will be appreciated by those of ordinary skill in the art from a reading of the figures and the detailed description of the preferred embodiments that follow, such description being merely illustrative of the present invention. Features described with respect with one embodiment can be incorporated with other embodiments although not specifically discussed therewith. That is, it is noted that aspects of the invention described with respect to one embodiment, may be incorporated in a different embodiment although not specifically described relative thereto. That is, all embodiments and/or features of any embodiment can be combined in any way and/or combination, provided such a combination is covered by the appended claims. The foregoing and other aspects of the present invention are explained in detail in the specification set forth below.

As will be appreciated by those of skill in the art in light of the present disclosure, embodiments of the present invention may include methods, systems, apparatus and/or computer program products or combinations thereof.

## Brief Description of the Figures

**Figure 1** is a flow chart of exemplary operations that can be used to calculate NMR measures of at least one BCAA according to embodiments of the present invention.

**Figure 2** is an NMR spectrum showing an example of a quantitative fitting of valine, isoleucine, and leucine according to embodiments of the present invention.

**Figures 3A-I** are graphs of exemplary linear concentration conversion models for BCAAs according to embodiments of the present invention.

Figure 4 is an expansion of an $^1$H NMR spectrum about a target branched chain amino acid methyl region according to embodiments of the present invention.

Figure 5 shows exemplary fitting for three BCAAs as an expansion of respective defined peak regions in the same NMR spectrum according to embodiments of the present invention.

Figure 6 is an exemplary pulse sequence using a standard "one-pulse" protocol with WET solvent suppression according to embodiments of the present invention.

Figure 7 illustrates the first pulse sequence in Figure 6 modified to include a CPMG sequence to attenuate signals associated with macromolecules and large aggregates such as proteins and lipoproteins according to particular embodiments of the present invention.

Figure 8 is an array of CPMG acquired $^1$H NMR spectra with respective different duration delay times.

Figure 9 is an expansion of the array of spectra of Figure 8 according to embodiments of the present invention.

Figure 10 is a schematic illustration of a system with a BCAA evaluation module and/or circuit using according to embodiments of the present invention.

Figure 11 is a schematic illustration of a NMR spectroscopy apparatus according to embodiments of the present invention.

Figure 12 is a schematic diagram of a data processing system according to embodiments of the present invention.

Figure 13 is a flow chart of exemplary operations that can be used to calculate NMR measures of BCAA according to embodiments of the present invention.

Figure 14A is a flow diagram of an NMR valine test protocol according to embodiments of the present invention.

Figure 14B is a flow chart of exemplary pre-analytical processing that can be used prior to obtaining NMR signal of biosamples according to embodiments of the present invention.

Figure 14C is a flow diagram of operations that can be used to evaluate valine using NMR according to embodiments of the present invention.

The foregoing and other objects and aspects of the present invention are explained in detail in the specification set forth below.

## Detailed Description of Embodiments of the Invention

The present invention now is described more fully hereinafter with reference to the accompanying drawings, in which embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

Like numbers refer to like elements throughout. In the figures, the thickness of certain lines, layers, components, elements or features may be exaggerated for clarity. Broken lines illustrate optional features or operations unless specified otherwise.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. As used herein, phrases such as "between X and Y" and "between about X and Y" should be interpreted to include X and Y. As used herein, phrases such as "between about X and Y" mean "between about X and about Y." As used herein, phrases such as "from about X to Y" mean "from about X to about Y."

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity. In case of a conflict in terminology, the present specification is controlling.

It will be understood that, although the terms first, second, etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention. The sequence of operations (or steps) is not limited to the order presented in the claims or figures unless specifically indicated otherwise.

The term "programmatically" means carried out using computer program and/or software, processor or Application Specific Integrated Circuit (ASIC) directed operations. The term "electronic" and derivatives thereof refer to automated or semi-automated operations carried out using devices with electrical circuits and/or modules rather than via mental steps and typically refers to operations that are carried out programmatically. The terms "automated" and "automatic" means that the operations can be carried out with minimal or no manual labor or input. The term "semi-automated" refers to allowing operators some input or activation, but the calculations and signal acquisition as well as the calculation of the concentrations of the ionized constituent(s) is done electronically, typically programmatically, without requiring manual input.

The term "about" as used herein refers to +/- 10% (mean or average) of a specified value or number.

"Branched chain amino acid" and "BCAA" as used herein refers to at least one of the following amino acids: valine, leucine, and isoleucine. As is known to those of skill in the art, branched chain amino acids are amino acids having a branched alkyl group side-chain. Valine has the chemical formula: $HO_2CCH(NH_2)CH(CH_3)_2$. Leucine has the chemical formula: $HO_2CCH(NH_2)CH_2CH(CH_3)_2$. Isoleucine has the chemical formula: $HO_2CCH(NH_2)CH(CH_3)CH_2CH_3$.

When a BCAA is measured by NMR, the value can be in a unitless measure of intensity. The intensity scale is constant such that the intensity of signals from different samples obtained under identical conditions can be directly compared. The intensities can be correlated to concentration. The BCAA unitless measurement may be multiplied by a defined conversion factor using calibration data to convert or translate the unitless value into concentration units, such as $\mu$mol/L concentration units.

The BCAA measurements may need to be adjusted based on an experimental condition before the fitting process. That is, the evaluation of the actual concentration of one or more BCAAs may be based on intensity, line width, and/or lineshape of respective samples which may be evaluated under different experimental conditions which may induce a variation or deviation in NMR spectrum from a standard or baseline experimental condition(s). One or more experimental conditions can be automatically or electronically determined *in situ* and/or proximate in time to acquisition of NMR data of respective samples for assessing one or more actual experimental conditions to allow a customization of a deconvolution model used, sample to sample..

The term "experimental condition" as used herein, can include, but is not limited to, the sample pH, the degree of water suppression, BCAA peak attenuation associated with a pulse *sequence* (such as a CPMG delay), the homogeneity of the magnetic field, sample preparation (*e.g.*, proper mixing of the sample and/or addition of the sample into the flow cell so that it completely fills the flow cell), the shim state of the instrument, and any combination thereof.

The terms" CAD" and "CHD" are used interchangeably to correspond to a patient or subject's risk of developing or having coronary artery and/or coronary heart disease, respectively. The term cardio vascular disease (CVD) refers to a combined outcome that is typically CHD plus stroke.

As used herein, the chemical shift locations (ppm) refer to NMR spectra referenced internally to CaEDTA signal at 2.519 ppm. Thus, the noted peak locations discussed and/or claimed herein may vary depending on how the chemical shift is generated or referenced as is well known to those of skill in the art. Thus, to be clear, certain of the described and/or claimed peak locations have equivalent different peak locations in other corresponding chemical shifts as is well known to those of skill in the art.

The term "biosample" refers to *in vitro* blood, plasma, serum, CSF, saliva, lavage, sputum, urine, or tissue samples of humans or animals. However, it should be noted that the invention is directed to an analysis of unfiltered *in vitro* serum or plasma samples.

The biosample may be unprocessed. An "unprocessed biosample" as used herein refers to a biosample that, unlike sample preparation for mass spectrometry analysis, is not subjected to processing that removes components in the biosample after it is obtained. Thus, once the biosample is obtained from a human or animal, components from the biosample are not removed. For example, once a blood serum biosample is obtained, the serum is not subjected to processing that removes components from the serum. In some embodiments, an unprocessed biosample is not subjected to filtering and/or ultrafiltration processes. An example of an unprocessed biosample is an unfiltered biosample that contains lipoprotein and protein components and a full complement of small molecule metabolites.

As those skilled in the art will recognize, various additives, standards, and/or diluents known to those of skill in the art may be added to the biosample to aid in the identification and/or quantification of components, such as BCAAs, in the biosample and/or to provide an experimental control. However, the addition of additives, standards, and/or diluents to a biosample does not refer to or encompass processing that causes the components in the biosample to be removed after the biosample is obtained. In certain embodiments, the biosample is an unfiltered biosample and is diluted with a diluent.

A diluent may be added to the biosample to form a sample mixture. Suitable diluents include, but are not limited to, buffer solutions and/or reference or calibration analytes, such as those known to those of skill in the art. Exemplary buffer solutions include, but are not limited to, acidic buffers, such as, but not limited to, a citrate, a phosphate, and/or an acetate buffer. In some embodiments, the diluent may comprise a pH buffer. The term "pH buffer" refers to a chemical or set of chemicals added to the biosample to create a defined pH-induced NMR peak shift in the NMR spectrum. Exemplary pH buffers include, but are not limited to, citrate phosphate buffers (e.g., citric acid and sodium dibasic phosphate, e.g., $C_6H_8O_7 \cdot H_2O$ and $Na_2HPO_4 \cdot 7H_2O$). For example, the biosample can comprise a citrate buffer and/or citrate phosphate buffer having a pH from about 2.6 to about 5.5 and comprising citric acid, sodium citrate, and/or sodium phosphate. Other exemplary buffers include, but are not limited to, an acetate buffer and/or an acetate phosphate buffer having a pH from about 3.7 to about 5.5 and comprising acetic acid, sodium acetate, and/or sodium phosphate. In some embodiments, the biosample comprises a citrate phosphate buffer comprising citric acid and sodium dibasic phosphate. Typically, a pH buffer is mixed with deionized water and added to the biosample to dilute the sample by a defined amount. However, pH buffer(s), where used, may also be added directly into a liquid or tissue biosample. In some embodiments, the NMR assay is performed on a sample mixture (e.g., the biosample and the diluent or diluted biosample) that may have a pH of between 4-10, such as, but not limited to, a pH of between about 7-8 or a pH of between about 7.2-7.6.

A biosample, diluent, and/or buffer may contain one or more endogenous and/or exogenous reference analyte(s) including, but not limited to, one or more of albumin, glucose, citrate, acetate or other acidic compounds as well any of the well established chemical shift or quantitation reference analytes such as formate, trimethylsilylpropionate (and isotopically labeled isomers), 4,4-dimethyl-4-silapentane-1-sulfonic acid (DSS), CaEDTA, and EDTA, for example.

In some embodiments, a biosample contains at least one endogenous or exogenous reference analyte. For example, a reference analyte can generate a reference peak or peaks associated with an endogenous or exogenous analyte. An example of an endogenous reference analyte is by way of example, but not limited to, glucose, that is present in the biosample. In some embodiments, the reference analyte has a chemical shift or quantitation peak(s) that is/are pH stable, meaning that the peak region and/or linewidth of the reference peak(s) over a pH range of between 4-10 does not substantially vary with pH (e.g., the peak region varies by less than about ± 0.1 ppm) or varies in chemical shift location and/or in size a known or predictable manner in response to changes in pH.

In some embodiments, the diluent may comprise or be a reference or calibration analyte, such as, but not limited to, CaEDTA and/or citrate.

The sample mixture may comprise a biosample to diluent ratio in a range of 10:90 to 90:10. In certain embodiments, the biosample to diluent ratio is 50:50. Buffers and/or other additives, e.g., reference or calibration analytes, may be added to the sample mixture. In certain embodiments, a buffer may maximize the amount of biosample in the mixture (e.g., provide a biosample to diluent buffer ratio of 55:45 or greater). It is contemplated that, in some embodiments, low volume biosamples may be analyzed, such as ≤ 50 μL or between about 10 μL to about 50 μL.

The term "patient" is used broadly and refers to an individual, human or animal, that provides a biosample for testing or analysis.

The term "clinical disease state" means an at-risk medical condition that may indicate medical intervention, therapy, therapy adjustment or exclusion of a certain therapy (e.g., pharmaceutical drug) and/or monitoring is appropriate. Identification of a likelihood of a clinical disease state can allow a clinician to treat, delay or inhibit onset of the condition accordingly. Examples of clinical disease states include, but are not limited to, metabolic disorders such as diabetes, CHD, CVD, stroke, type 2 diabetes, prediabetes, liver disease, cancer, and cancer cachexia.

The term "CPMG" refers to a Carr-Purcel-Meiboom-Gill pulse sequence. This is a series of phase defined radiofrequency pulses that provide means to attenuate signals from large, rapidly relaxing molecules such as proteins and lipoprotein particles.

A single NMR spectrum is generated in response to a defined pulse sequence. For example, one NMR spectrum is obtained using a one-pulse sequence and/or one NMR spectrum is obtained using a CPMG pulse sequence. Typically, a plurality of scans per NMR spectrum, such as between about 16-96 scans, are used to acquire signal to generate the associated NMR spectrum using a signal averaging process so that signal has improved signal to noise (SNR).

Embodiments of the invention may provide a simple, fast and convenient assay for the quantification of one or more branched chain amino acids (BCAAs) in a biosample using NMR. In the assay, separate quantification of all three BCAAs may be accomplished using NMR spectra of an unfiltered sample, optionally with the use of a reference analyte for generating a reference peak(s). The sample can have an exogenous and/or endogenous reference analyte that provides at least one reference peak that can be evaluated for linewidth and/or lineshape that is associated with inhomogeneity of the Bo static magnetic field of the NMR spectrometer. The shim state or Bo homogeneity/inhomogeneity can be evaluated automatically for each sample or proximate in time to NMR acquisition time of a respective sample or set of samples using the reference peak(s).

The deconvolution model can be configured to select a computational fitting function (CFF) (e.g., curve fitting function) with appropriate linewidth and/or lineshape based on the reference peak(s) linewidth and/or lineshape as determined for each respective sample NMR spectrum for BCAA quantification.

Two or three BCAAs may be separately quantified in a biosample.

In certain embodiments, a NMR assay according to embodiments of the present invention may provide a composite measure of three BCAAs present in a biosample to generate a composite unitless score or concentration measure of valine, isoleucine, and leucine versus separate discrete scores or concentrations.

The quantified concentration measurements for one or more of the BCAAs, e.g., at least valine and leucine, can be carried out to provide measurements with spiked dialyzed standards, as shown below in **Table 1.** It is contemplated that the calibration data can provide concentration measurements, with measured percent coefficient of variances (CV) (% CVs) of less than 15% at the mean concentrations in a healthy population.

**Table 1:** Spiked dialyzed BCAA measurements.

| spike | NMR Leucine | NMR Leucine | spike | NMR Valine | NMR Valine | spike | NMR Isoleucine | NMR Isoleucine |
|---|---|---|---|---|---|---|---|---|
| 0 | 98.92 | 9.62 | 0 | 117.44 | 12.36 | 0 | 32.36 | 13.23 |
| 60 | 173.74 | 3.48 | 80 | 212.07 | 4.41 | 10 | 56.45 | 13.74 |
| 120 | 251.42 | 2.96 | 160 | 290.18 | 7.72 | 20 | 96.34 | 8.18 |
| 180 | 323.92 | 5.41 | 240 | 372.03 | 6.01 | 30 | 140.75 | 10.41 |
| 240 | 393.37 | 1.71 | 320 | 466.03 | 7.81 | 40 | 181.96 | 8.01 |

| 98.7 +/- 11.5 | 212.3 +/- 62.3 | 60.7 +/- 18.6 |
|---|---|---|

BCAAs may be central components of energy metabolism. In some embodiments, a NMR assay according to embodiments of the present invention may provide a ratio of at least one BCAA to at least one BCAA or to a group of BCAAs. The ratio can be generated based on a different combination of defined BCAAs, and thus the combination may include one or more BCAAs present in the sample. Alternatively or in addition, a NMR assay according to embodiments of the present invention may provide a ratio of one or more BCAAs to another metabolite. The ratios may have diagnostic value and may be used in evaluating a patient's clinical disease state and/or provide metabolic status or other therapeutic or diagnostic information. For example, a ratio of at least one BCAA to at least one aromatic amino acid (*e.g.,* phenylalanine, tyrosine, tryptophan, and histidine) may be obtained according to embodiments of the present invention, and may be used to predict and/or diagnose a liver disease and/or provide information for treating a patient diagnosed with a liver disease. In some embodiments, a Fischer's ratio (*i.e.,* the sum of all three BCAAs to the sum of phenylalanine and tyrosine in the biosample) may be calculated according to embodiments of the present invention. Ratios of at least one BCAA with other exemplary metabolites include, but are not limited to, metabolites such as those participating in glycolysis and/or b-oxidation pathways. In some embodiments, a ratio with a BCAA component may be electronically calculated or obtained.

Identification and quantification of a particular BCAA in a biosample may be accomplished according to embodiments of the invention. In some embodiments, at least three BCAAs (*i.e.,* valine, leucine, and isoleucine) are identified in respective biosamples and each of the three BCAAs in the biosample can be separately quantified using a single NMR spectrum.

Alternatively or in addition, a composite score or concentration of the BCAAs can be generated.

In some embodiments, a weighted index, ratio or ratio with a weighted index having at least one of the unitless signal or concentrations of BCAAs can be calculated. The ratio and/or weighted index can be generated based on a regression analysis of outcomes for respective diseases that is predictive of a clinical outcome or disease risk or identifies an efficacy of a drug or drug candidate, The weighted index can weight one or more BCAA unitless signals or measurements more than another BCAA component or may multiply certain other risk components together forming a patient health or risk indicator score or index. In the below examples, "V" refers to valine, "IL" refers to isoleucine, and "L" refers to leucine. Ratios include, but are not limited to: V/(L+IL); I/IL; V/L; IL/V; V/(IL); (VL)/IL and the like. Weighted indexes include, but are not limited to: V*GlycA/V(IL)(L): IL/L(10)+V;V(L)(IL)/Lx10 and V/(ILx10), for example. _ :

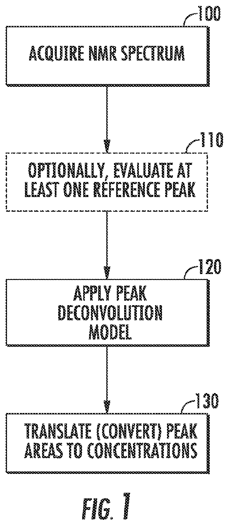

According to some embodiments of the present invention, a method may comprise acquiring an NMR spectrum of an unfiltered sample using a pulse sequence (**Figure 1,** block **100**). The pulse sequence may suppress or attenuate macromolecule signals (*e.g.,* lipoprotein and protein signals) in the spectrum, optionally, the pulse sequence may be optimized. The term "optimized", as used herein, means that the pulse sequence is configured so that macromolecule signals are attenuated in the NMR spectrum to remove or reduce interference with small molecule BCAAs to a sufficient degree to allow for the quantification of one or more BCAAs.

The suppression of macromolecular signals may be carried out in a manner that suppresses small molecule (*i.e.,* compounds or molecules with a molecular weight of up to about 500 Daltons or less) signals by up to 40% (*e.g.,* typically between about 2% to about 30%). For at least the larger attenuations, the suppression can be generated to yield predictable attenuations that can optionally be adjusted for using one or more quantification adjustment factors and/or models.

In certain embodiments, the NMR spectrum with a pulse sequence can generate a lipoprotein and/or protein signals and these signals may be accounted for computationally in a manner that allows for quantification of BCAAs.

Optionally, one or more reference peaks (*e.g.,* 2, 3, 4, or more) may be identified and/or evaluated to identify and/or quantify a target BCAA peak or peaks for a respective BCAA (block **110**). The reference peak(s) may be associated with an endogenous and/or exogenous reference analyte, which may be a pH chemical-shift invariant reference peak. A pH chemical-shift "invariant" reference peak is a peak that is pH insensitive over a pH of between about 4 to about 10 and has a chemical shift location that is within +/- 0.1 ppm.

The one or more reference peaks may be electronically evaluated to determine their location, lineshape, and/or linewidth. The one or more reference peaks may be used to determine an experimental condition, such as, but not limited to, sample pH and/or magnetic field inhomogeneity. The one or more reference peaks may be determined for each sample and used to evaluate each sample NMR spectrum. The one or more reference peaks may thus be used to select a defined computational (e.g., curve) fitting function, linewidth of a CFF, and/or for determining the location of one or more BCAA peaks and/or the measurement/concentration of a respective BCAA.

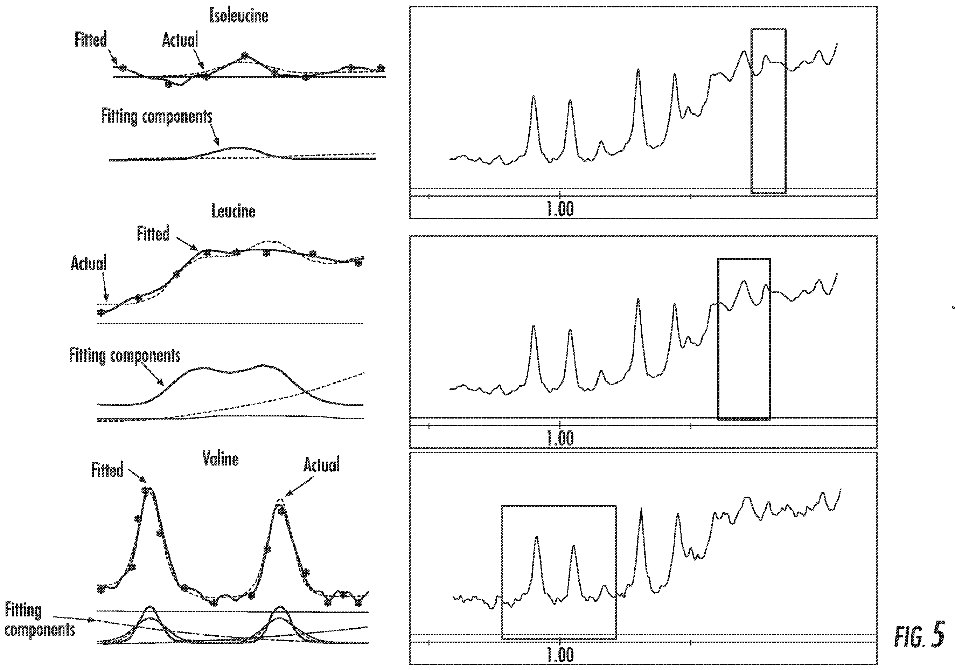

A computational curve fitting model is used to deconvolve the NMR spectrum (block **120**). The model may include defined mathematical deconvolution functions. The model may include fitting functions with determined lineshapes and/or linewidths to a targeted BCAA peak position. The model may include computational fitting functions with baseline contributions, such as, for example, as shown in **Figure 5.** The fitting functions can be any suitable function such as Lorenztians or Gaussians.

In some embodiments, the fitting model may involve using scalar coupling of the BCAAs. Scalar coupling is the spectroscopic phenomenon wherein a chemical species gives rise to multiple peaks which are separated by a consistent distance as measured in Hertz, irrespective of magnetic field strength. The fitting functions can be constrained to a separation distance at or very near this distance in order to constrain the fitting process.

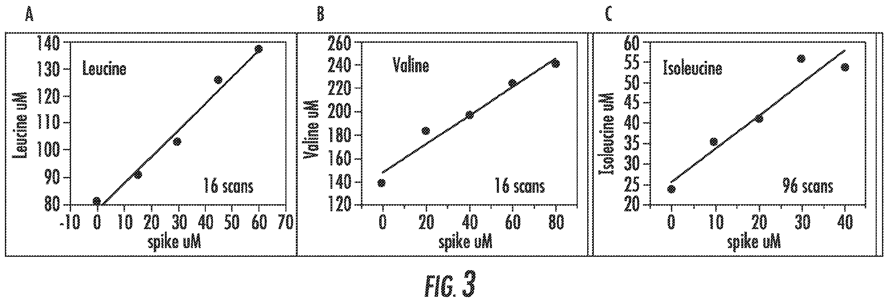

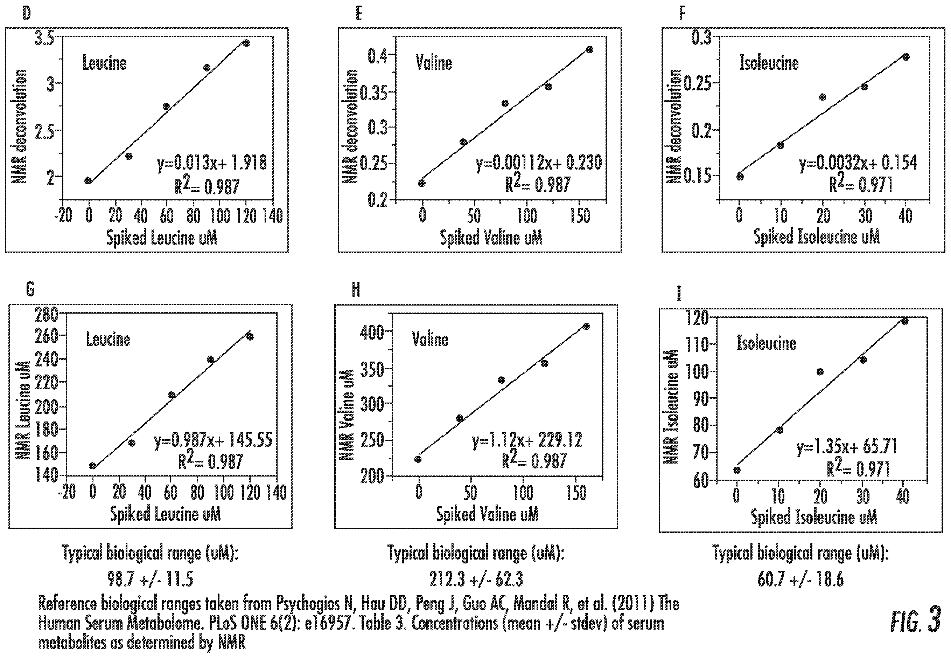

A BCAA concentration may then be determined based on a BCAA peak area (block **130**). Concentration conversion data may be obtained from linear mathematical equations or associated calibration lines or graphs or look-up tables or the like, may be used to determine the respective BCAA concentration. The calibration spectra are acquired under spectroscopic conditions which are identical to those used in the final assay, such as that shown in **Figures 3A-I**. As shown in **Figures 3A-I**, typical biological ranges for leucine, valine, and isoleucine are about 98.7 $\pm$ 11.5 $\mu$M, 212.3 $\pm$ 62.3 $\mu$M and 60.7 $\pm$ 18.6 $\mu$M, respectively. In some embodiments, the calibration data may account for small differences in nuclear relaxation properties, typically less than 10%.

A respective single BCAA measurement or concentration may be used alone or in combination with another BCAA measurement or concentration or other metabolite measurement or concentration to evaluate a subject's clinical disease state and/or provide metabolic status or other therapeutic or diagnostic information. In some embodiments, a regression model may be used to correlate BCAA components into a composite BCAA number or score, weighted index and/or a BCAA ratio of different BCAA components with a particular therapeutic or diagnostic outcome.

In some embodiments, a pulse sequence can be configured to attenuate lipoproteins and proteins in an unfiltered biosample. One example is a CPMG sequence. Identification and/or quantification of other components (*e.g.,* metabolites) in the biosample, such as, but not limited to, other amino acids (*e.g.,* aromatic amino acids), organic acids, 2-hydroxybutyrate, GlycA, GlycB, and/or lipoproteins may also be accomplished according to embodiments of the present invention.

In some embodiments, identification and/or quantification of a BCAA and an aromatic amino acid may be carried out with a sample mixture at a pH of about 7 or about 7.4.

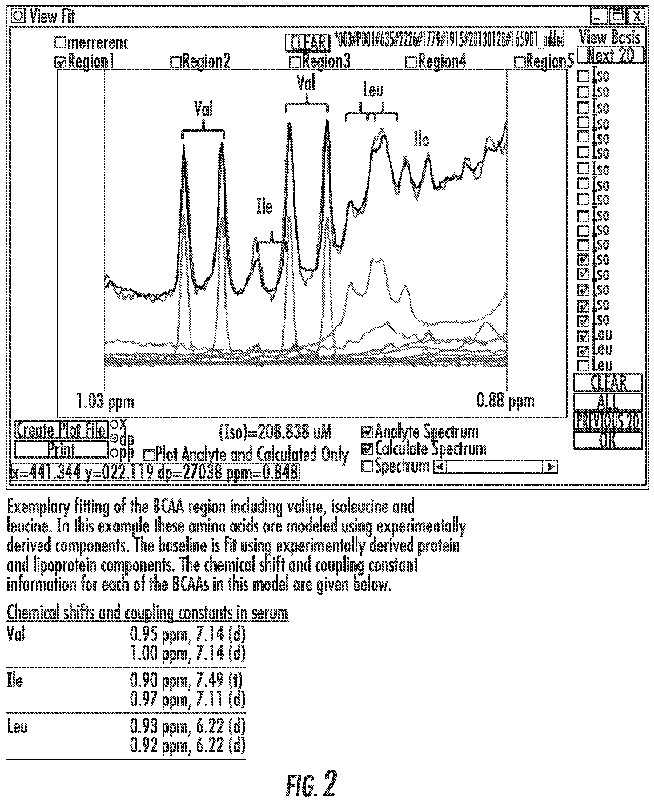

The BCAA peak region may fall between about 0.85 ppm and about 1.10 ppm (at about 47°C sample temperature). The peak locations for valine, leucine, and isoleucine, at about 47°C sample temperature, may be as listed in the table in **Figure 2.**

In modeling the BCAA region of the NMR spectrum, an experimental and/or synthetic model may be used for a BCAA. The baseline may be modeled by CPMG processed experimental spectra of the individual protein and lipoprotein components that are known to contribute to this region. The model may also use synthetic functions such as, but not limited to, linear, quadratic and polynomial functions to fit the baseline component. The fitting approach may utilize the Lawson-Hanson non-negative linear least fitting method to achieve the best agreement between the experimental and modeled spectra.

**Figures 3A-I** show exemplary analytical models for BCAAs. In this example, a 100 ms delay CPMG pulse sequence was used. As can be seen in **Figures 3A-C**, the analytical models for leucine and valine were based on 16 scans and isoleucine was based on 96 scans. **Figures 3D-F** show the raw area NMR values for valine, leucine, and isoleucine based on 96 scans and **Figures 3G-I** show the converted NMR concentrations ($\mu$M) for the respective valine, leucine, and isoleucine values in **Figures 3D-F**. The concentrations can be calculated for normal biological ranges appropriating norms for each BCAA. Typical biological ranges for leucine, valine, and isoleucine are 98.7 $\pm$ 11.5 $\mu$M, 212.3 $\pm$ 62.3 $\mu$M and 60.7 $\pm$ 18.6 $\mu$M, respectively, as described in Psychogios et al. (2011) The Human Serum Metabolome. PLoS ONE 6(2): e16957, Table 3. Concentrations (mean +/- standard deviation) of serum metabolites as determined by NMR.

According to some embodiments of the present invention, separate quantification of at least three BCAAs present in an unfiltered biosample may be accomplished without the need for an internal or external reference analyte.

Accurate quantification of BCAAs in an unfiltered biosample may be affected by Bo homogeneities/inhomogeneities associated with the shimming of the spectrometer. Thus, in some embodiments, quantification may be carried out by selecting a deconvolution model with a CFF or a linewidth thereof on a sample to sample basis. For example, for each sample a linewidth and/or line shape of a defined reference peak(s) in the spectrum can be evaluated. The reference peak(s) can include, but is not limited to, an EDTA peak and/or citrate peak.

A signature of at least one branched chain amino acids may be obtained according to embodiments of the present invention. An exemplary signature of all three branched chain amino acids is shown in **Figure 2,** which shows the fitting of the BCAA region of the spectrum. The signature shown in **Figure 2** was obtained from a 75:25 mixture of serum to citrate buffer (*i.e.,* diluent). The data was collected using a 16 scan CPMG experiment with an acquisition time of approximately 90 seconds and a 100 ms CPMG pulse train. The model included computationally derived basis functions for each of the three BCAAs as well as experimental protein and lipoprotein functions. The identification and/or quantification of signals of metabolites, including amino acids and organic acids, may also be obtained from such an experiment. According to some embodiments of the present invention, an experiment for the quantification of BCAAs as described herein may be appended to a standard Lipoprofile® lipoprotein analysis for high-throughput analysis of respective biosamples.

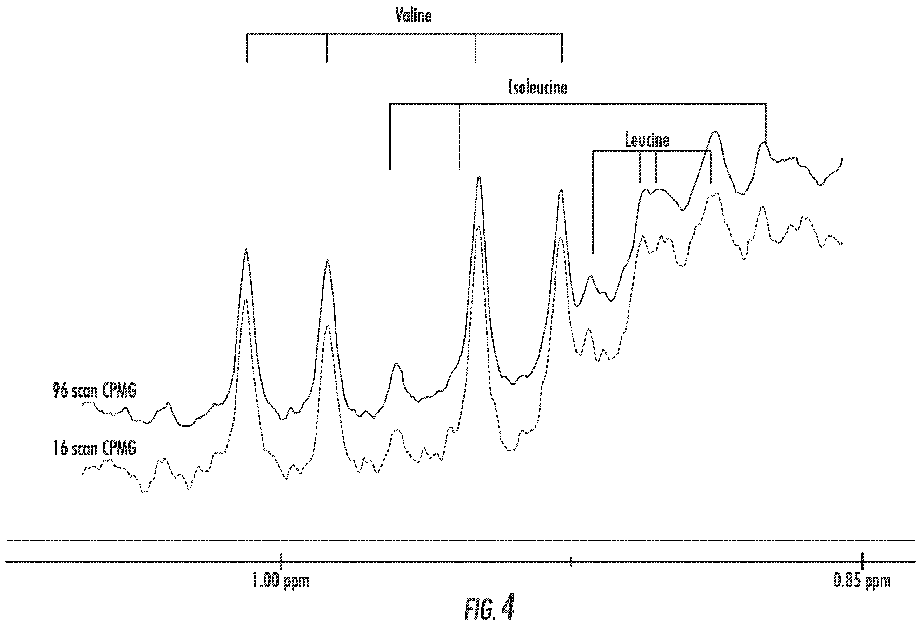

**Figure 4** shows an expansion of a $^1$H NMR spectrum about the branched chain amino acid methyl region. The top spectrum was acquired with 96 scans and the bottom spectrum was acquired with 16 scans. In some embodiments, concentrations of leucine and valine can be calculated using less scans than isoleucine. Each BCAA can be calculated using the same NMR spectrum. Each BCAA can be evaluated using the same number of scans or a different number of scans.

Quantified measures of a BCAA in a biosample according to embodiments of the present invention may be used to determine a likelihood of a patient having or developing one or more defined clinical disease states. BCAAs may be associated with metabolic diseases, such as diabetes (*e.g.,* type 2 diabetes), CHD, liver diseases, and cancer. Accordingly, an assay of the present invention may be used to determine a patient's clinical disease state. In some embodiments, an assay according to embodiments of the present invention may be used to determine a patient's risk of developing a disease, such as, but not limited to a metabolic disease (*e.g.,* the patient's risk of incident diabetes).

According to some embodiments, an assay according to embodiments of the present invention may be used for disease prediction and/or intervention outcomes. In certain embodiments, the assay may be used as a predictor of improved insulin sensitivity in a patient and/or to determine if there has been an improvement in glycemic control in a patient. In other embodiments, the assay may be used in determining if there has been an enhancement in liver regeneration in a patient or attenuation in muscle wasting in a patient, such as a cancer patient. In some embodiments, the assay may be used to determine the potential benefit and/or need for BCAA supplementation in a patient diagnosed with a wasting syndrome and/or severe inflammatory diseases (*e.g.,* sepsis, trauma, burn injury, etc.). In further embodiments, the assay may be used to guide and/or monitor the use of BCAAs in nutritional supplementation and/or improvement of health, wellness and/or athletic performance.

A defined mathematical deconvolution model can be used to quantify a BCAA. The "composite" or measured signal envelope Cm can be deconvolved to quantify the signal contributions of a BCAA and other contributing components such as lipoprotein subclass components. The deconvolution calculates signal amplitudes of the components contributing to the measured signal shapes and calculates the sum of the components. A close match between the calculated signal C and the measured signal Cm indicates the deconvolution successfully modeled the components that make up the NMR signal.

A curve fitting function or technique can be used according to embodiments of the present invention. The curve fitting may use different sets of basis functions that can vary biosample to biosample, which can include none, or one or more neighboring BCAA peaks that reside adjacent to a target BCAA peak.

The lineshape deconvolution can be achieved with a non-negative least squares fitting program (Lawson, CL, Hanson RJ, Solving Least Squares Problems, Englewood Cliffs, NJ, Prentice-Hall, 1974). This avoids the use of negative concentrations which will lead to error due especially in low signal to noise spectra. Mathematically, the lineshape analysis is described in detail for lipoproteins in the paper by Otvos, JD, Jeyarajah, EJ and Bennett, DW, Clin Chem, 37, 377, 1991. A synthetic baseline correction function may also be used to account for baseline offsets from residual protein components. This can take the form of a quadratic or other polynomial function. Weighting factors are determined and the fit can be optimized by minimizing the root mean squared deviation between the experimental and calculated spectrum. *See, e.g.,* U.S. Patent Nos. U.S. Patent Nos. 4,933,844 and 6,617,167 for a description of deconvolving composite NMR spectra to measure subclasses of lipoproteins. *See also,* U.S. Patent No. 7,243,030 for a description of a protocol to deconvolve chemical constituents with overlapping signal contribution.

**Figure 5** shows exemplary computational fitting for the three BCAAs. The panels on the left show the respective BCAA region from the expansions of the $^1$H NMR spectral region about the branched amino acid methyl signals from the NMR spectrum on the right. For each amino acid a target peak or peaks for quantification have been boxed. The panels on the left show the "actual" spectrum (black), the "fitted" spectrum (light grey) with the associated fitting components (below). The fitting components (*e.g.,* gaussians or lorentzians or both) may contain experimental and/or computationally derived peaks and/or functions that model the contributions of the target analyte as well as the contributions from sources such as proteins, lipoproteins and known spectrometer induced offsets. In some embodiments, the fitting components may contain experimental and computationally derived peaks and functions that model the contributions of the target analyte as well as the contributions from sources such as proteins and lipoproteins. The factors and models may also provide for spectrometer induced offsets known to those of skill in the art such as DC offsets.

The pulse sequence parameters can include any appropriate parameters including solvent suppression scheme, pulse angle and acquisition time. However, generally stated, in some particular embodiments, the NMR signal acquisition time per scan, for any one biosample, can be between about 2-7 seconds (on average) and typically between about 5-6 seconds (on average), such as about 5.6 seconds (on average). In some embodiments, the total acquisition time is about 90 seconds (on average) or up to about 5 or 10 minutes (on average). The NMR analyzer may be configured to obtain at least 10 scans per biosample, typically between 10-256 scans, such as 16 scans, and possibly $\geq 96$, such as 96 scans or 128 scans with at least about 16K data points collected over a 4400 Hz sweep width, per sample, to obtain the NMR data used to measure BCAA. The BCAA scan can be carried out before or after a lipoprotein scan sequence, which typically employs a different pulse sequence to allow for quantification of lipoproteins.

In some embodiments, one element in the pulse sequence can be a solvent suppression scheme. A WET solvent suppression scheme uses a series of shaped pulses and pulsed field gradients over the course of, for example, about 80 ms. The ID NOESY-presat scheme uses the first increment of a 2D Nuclear Overhauser Effect Spectroscopy (NOESY) experiment (Beckonert, O.; Keun, H. C.; Ebbels, T. M. et. al. Nat. Protoc. 2007, 2, 2692-2703). In this scheme, a continuous low power, frequency selective pulse on water resonance is applied during D1 and 'mixing' time. The PURGE solvent suppression scheme (Simpson, A. J.; Brown, S. A. J. Magn. Reson. 2005, 175, 340-346) uses a continuous low power, frequency selective pulse on water resonance, relaxation gradients and echoes to attenuate the water signal.

The performance of all three sequences (and potentially other sequences known to those of skill in the art) is sufficient to achieve consistent solvent suppression in the spectra. Stated differently, a BCAA NMR signal may be obtained using CPMG and a WET, PRESAT, and/or PURGE protocol may be included as part of a pulse sequence. One advantage of the WET sequence is that it does not involve any low power saturation period which could perturb the protein baseline via spin diffusion. It also does not have any significant delays which could lead to signal attenuation via relaxation.

As is well known, a standard presaturation ("Presat") pulse sequence can be used in a pulse sequence to obtain the NMR spectrum for analyzing the BCAA signal. This pulse sequence involves a selective low power pulse targeting the water resonance and lasting several seconds. This is well established in NMR practice and is a robust and reliable method to attenuate the water signal.

In some embodiments, the WET water suppression scheme can be used. The WET sequence involves a series of short selective pulses targeting the water resonance. The entire scheme is prefixed to the pulse sequence as is the Presat, but only requires, for example, about 80 ms. The other advantage of the WET sequence is the fact that this sequence imposes only a minimal perturbation on the protein signals. Due to the length of a typical Presat sequence, some of the solvent saturation can be transferred to the protein which can lead to inconsistent contributions of the protein to the baseline. Other solvent presaturation schemes can be used, e.g., a PURGE sequence.

The flowcharts and block diagrams of certain of the figures herein illustrate the architecture, functionality, and operation of possible implementations of analysis models and evaluation systems and/or programs according to the present invention. In this regard, each block in the flow charts or block diagrams represents a module, segment, operation, or portion of code, which comprises one or more executable instructions for implementing the specified logical function(s). It should also be noted that in some alternative implementations, the functions noted in the blocks might occur out of the order noted in the figures. For example, two blocks shown in succession may in fact be executed substantially concurrently or the blocks may sometimes be executed in the reverse order, depending upon the functionality involved.

**Figure 6** illustrates a standard "one-pulse" sequence with WET solvent suppression. This leads to a spectrum in which the signals from the high concentrations of proteins and macromolecular aggregates, e.g. lipoprotein particles, dominate the spectra. The lower concentration, small molecule metabolites are greatly obscured in these spectra. As shown in **Figure 6,** the WET solvent suppression scheme (Smallcombe, S. H.; Patt, S. L.; Keifer, P. A. J. Magn. Reson., Ser. A 1995, 117, 295-303) includes a series of solvent-directed selective pulses and pulsed field gradients followed by a read pulse and an acquisition time during which the signal is digitized for a fixed amount of time.

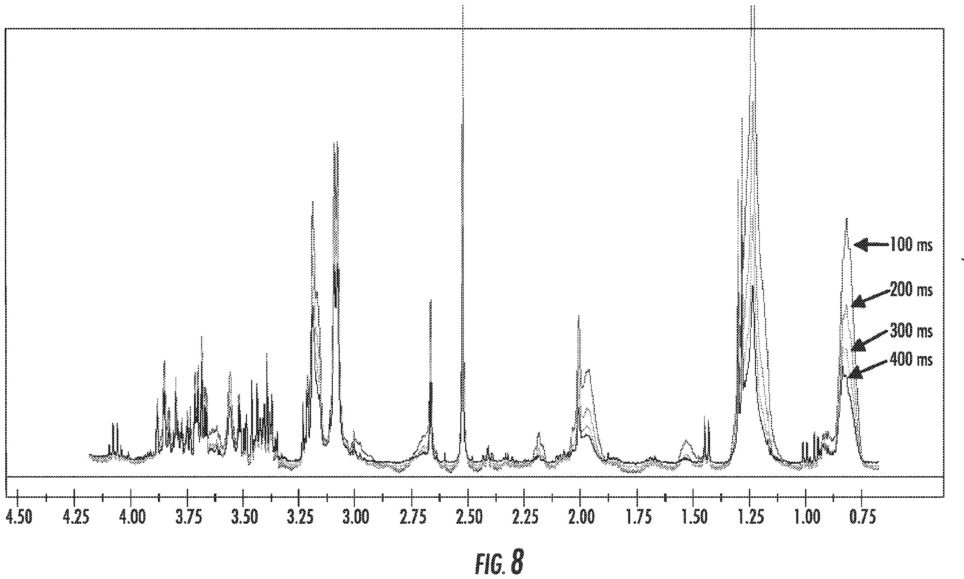

The second sequence shown in **Figure 7** incorporates a CPMG pulse train or sequence. This pulse train includes a series of refocusing π pulses (180 degrees) during which the signals from large, rapidly relaxing molecules are attenuated. The duration of this pulse train can be optimized to minimize the background signals from macromolecules, e.g. proteins and lipoprotein particles, while maintaining most of the signal intensity from the small molecules such as BCAA. This value is set to 100 ms, but other delays can be used. For example, CPMG pulse sequence delays may be set to 60 ms, 100 ms, 200 ms, 300 ms, or 400 ms. Thus, the CPMG pulse train shown in **Figure 7** is designed to attenuate the signals from the macromolecules which facilitate the detection of many more small molecule metabolites. This pulse sequence relies on the fact that the signals from macromolecules relax much faster than small molecules. The spin-echo train occurring can occur after the read pulse and is designed to maintain the small molecule magnetization while the signals from the macromolecules relax back to equilibrium. The duration of this spin echo train relates to the degree of attenuation of the macromolecule signals. This delay is typically set to between about 60 ms to about 400 ms (**Figure 8**). It is expected that there will be little perceptible change in performance over the range from about 60 ms to about 150 ms. In some cases this delay could be set to as high as about 400 ms if the benefit of increased attenuation of the proteins and lipoproteins offsets the reduced signal to noise. In some embodiments, attenuation of the BCAA NMR signal caused by the pulse sequence may be corrected using an adjustment factor.

In order to obtain an increased (e.g., maximum) signal from a molecule, a 90 degree pulse can be used and the time between these pulses should be in excess of 10 times the longitudinal relaxation time (T1) of the signal. The T1 for a BCAA at 400 MHz may be in a range of about 1.5 to about 3 seconds. This may not be a time efficient means to maximize the signal intensity so a compromise between pulse length and inter-pulse delay can be made. The relationship between signal intensity, T1, and pulse length is given by the Ernst angle equation. The first step in using this equation is to define the length of the entire pulse sequence. The length is defined by the required solvent suppression period, the CPMG delay and the length of the data acquisition period needed to provide the required digitization of the FID. The Ernst angle equation is as follows:

$$\mathrm{Cos}\,(\mathrm{theta}) = \exp{-(\mathrm{total\ delay})/\mathrm{T1}} \quad \textbf{EQUATION (1)}$$

In equation (1), the total delay equals the d1 delay (including solvent suppression), the CPMG time, plus the acquisition time. T1 represents the longitudinal relaxation time of the analyte signal of interest. It is noted that in front of the parentheses containing "total delay" is a negative sign. Solving for theta will give an optimal flip angle.

Using a standard set of experimental conditions, for example, a 1 second period for solvent suppression, 100ms CPMG, and an acquisition time of about 3 seconds, the Ernst angle may prescribe the tip angle (also known as the pulse length when described in microseconds) that is reduce from 90 degrees down to between 60 and 80 degrees. The equation is relatively insensitive in this region so it is unlikely that small errors in calibration or small differences in the T1 due to specific sample composition will lead to significant inefficiency.

The acquisition time (AT) is the time that the FID is digitized. The duration of AT is determined by both the relaxation time of the signal(s) being quantified and the required digitization. If the relaxation time of the signal being examined is longer than the AT then the FID will become truncated resulting in a signal with poor shape. As mentioned above, the T1 relaxation time for a BCAA may be less than about 3 seconds in serum and thus the acquisition time for a scan should be at least that long. The digitization rate of the spectrometer, i.e. the number of points taken per second of acquisition time, is determined by the sweep width of the spectrum. In some embodiments, a desired digital resolution uses at least 16K data points that are collected over the 4400Hz sweep width.

The most direct way to increase the detection sensitivity is to increase the number of scans. In some embodiments, the BCAA assay can be carried out on samples at about 47°C so that this assay can be easily interleaved or used with the current LipoProfile® assay from LipoScience, Inc. (Raleigh, NC). However, as the number of scans increases, the residence time of the sample in the probe at 47°C increases and the samples may become denatured. The challenge is to achieve the requisite signal-to-noise ratio for a BCAA peak to allow accurate and precise quantification over a desired biological range (at least those with adverse clinical association).

In some embodiments, the NMR assay may include a separate quantification for each BCAA and/or a total quantification of at least three of the BCAAs (*i.e.,* the quantified sum of the BCAAs) present in a biosample, and each of the quantified measurements may be provided in a patient report for clinical consideration. It is contemplated that the separate or total quantification of the BCAAs in a single *in vitro* biosample may provide important clinical information and/or further improve a prediction or evaluation of a patient's risk of having a clinical disease state or being at increased risk for a clinical disease state.

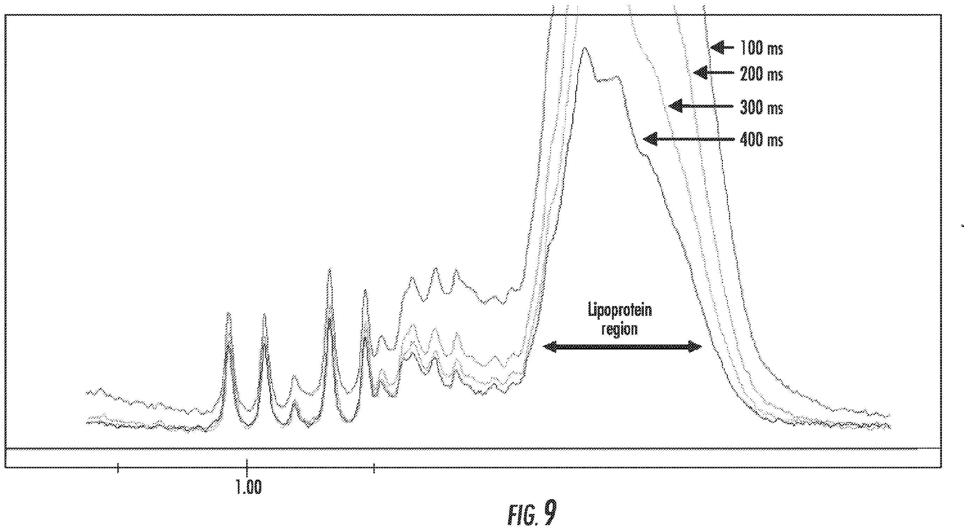

**Figure 8** shows an array of CPMG acquired $^1$H NMR spectra. The spectra may be acquired using the CPMG sequence which suppresses the contributions to the spectrum from macromolecules such as proteins and macromolecular aggregates such as lipoproteins. As shown in **Figure 7** the CMPG pulse sequence may involve a pulse train of variable delay during which the signals from the large molecules decay away thereby enabling the detection of more of the small molecule metabolites. The longer CPMG delay times may lead to greater attenuation of these signals. This delay may also attenuate the small molecule signals, but to a significantly lesser extent. **Figure 9** shows expansion of the array of CPMG spectra acquired with different total delay times. The expansion focuses on the methyl region of the BCAAs as previously shown in **Figure 4.** Notice that the signals in the lipoprotein region are significantly attenuated while the total intensity of the BCAA peaks is less affected.

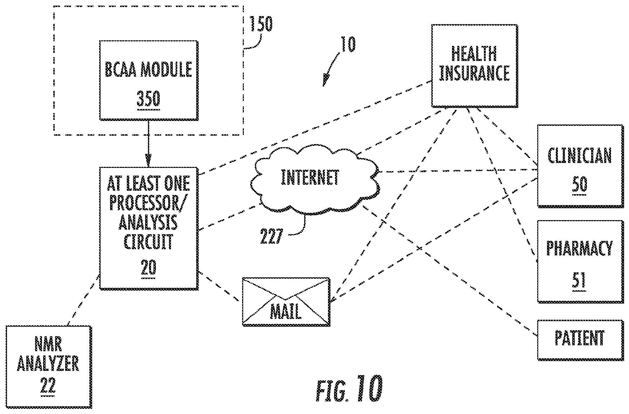

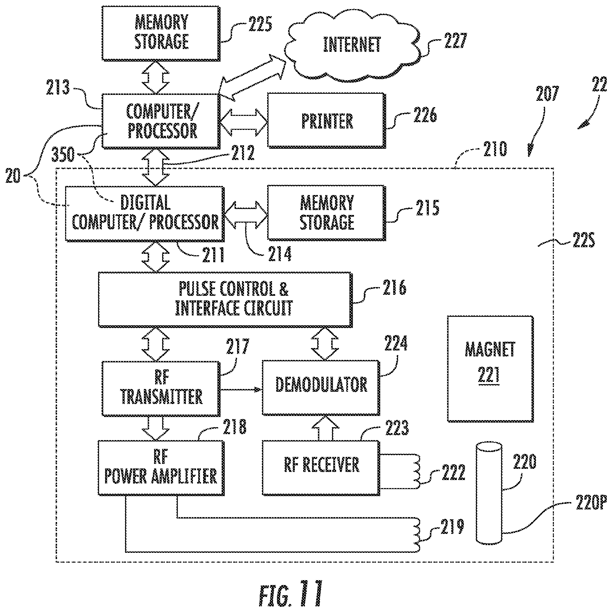

Referring now to **Figure 10,** it is contemplated that the quantification of a BCAA may be carried out using a system **10** with an NMR clinical analyzer **22** as described, for example, with respect to **Figure 11** below and/or in U.S. Patent No. 8,013,602. The analyzer **22** includes a spectrometer **22s** and sample handler system.

The system **10** can include a BCAA analysis module and/or circuit **20** that can be onboard the analyzer **22** or at least partially remote from the analyzer **22.** If the latter, the analysis module or circuit **20** can reside totally or partially on a server **150.** The server **150** can be provided using cloud computing which includes the provision of computational resources on demand via a computer network. The resources can be embodied as various infrastructure services (e.g. computer, storage, etc.) as well as applications, databases, file services, email, etc. In the traditional model of computing, both data and software are typically fully contained on the user's computer; in cloud computing, the user's computer may contain little software or data (perhaps an operating system and/or web browser), and may serve as little more than a display terminal for processes occurring on a network of external computers. A cloud computing service (or an aggregation of multiple cloud resources) may be generally referred to as the "Cloud". Cloud storage may include a model of networked computer data storage where data is stored on multiple virtual servers, rather than being hosted on one or more dedicated servers. Data transfer can be encrypted and can be done via the Internet using any appropriate firewalls to comply with industry or regulatory standards such as HIPAA. The term "HIPAA" refers to the United States laws defined by the Health Insurance Portability and Accountability Act. The patient data can include an accession number or identifier, gender, age and test data.

The results of the analysis can be transmitted via a computer network, such as the Internet, via email or the like to a patient, clinician site **50,** to a health insurance agency **52** or a pharmacy **51.** The results can be sent directly from the analysis site or may be sent indirectly. The results may be printed out and sent via conventional mail. This information can also be transmitted to pharmacies and/or medical insurance companies, or even patients that monitor for prescriptions or drug use that may result in an increase risk of an adverse event or to place a medical alert to prevent prescription of a contradicted pharmaceutical agent. The results can be sent to a patient via email to a "home" computer or to a pervasive computing device such as a smart phone or notepad and the like. The results can be as an email attachment of the overall report or as a text message alert, for example.

Referring now to **Figure 11,** a system **207** for acquiring and calculating the lineshape of a selected sample is illustrated. The system **207** includes an NMR spectrometer **22s** for taking NMR measurements of a sample. In one embodiment, the spectrometer **22s** is configured so that the NMR measurements are conducted at 400 MHz for proton signals; in other embodiments the measurements may be carried out at between 200 MHz to about 900 MHz or other suitable frequency. Other frequencies corresponding to a desired operational magnetic field strength may also be employed. Typically, a proton flow probe is installed, as is a temperature controller to maintain the sample temperature at 47 +/- 0.5 degrees C. However, other sample temperatures may be used during acquisition time. The spectrometer **22** is controlled by a digital computer **214** or other signal processing unit. The computer **211** should be capable of performing rapid Fourier transformations. It may also include a data link **212** to another processor or computer **213,** and a direct-memory-access channel **214** which can connect to a hard memory storage unit **215.**

The digital computer **211** may also include a set of analog-to-digital converters, digital-to-analog converters and slow device I/O ports which connect through a pulse control and interface circuit **216** to the operating elements of the spectrometer **22s.** These elements include an RF transmitter **217** which produces an RF excitation pulse of the duration, frequency and magnitude directed by at least one digital signal processor that can be onboard or in communication with the digital computer **211,** and an RF power amplifier **218** which amplifies the pulse and couples it to the RF transmit coil **219** that surrounds sample cell **220** and/or flow probe **220p.** The NMR signal produced by the excited sample in the presence of a 9.4 Tesla polarizing magnetic field produced by superconducting magnet **221** is received by a coil **222** and applied to an RF receiver **223.** The amplified and filtered NMR signal is demodulated at **224** and the resulting quadrature signals are applied to the interface circuit **216** where they are digitized and input through the digital computer **211.** The lipoprotein measurement and/or BCAA analyzer circuit **20** and/or module **350 (Figures 11-12)** can be located in one or more processors associated with the digital computer **211** and/or in a secondary computer **213** or other computers that may be on-site or remote, accessible via a worldwide network such as the Internet **227.**

After the NMR data are acquired from the sample in the measurement cell **220,** processing by the computer **211** produces another file that can, as desired, be stored in the storage **215.** This second file is a digital representation of the chemical shift spectrum and it is subsequently read out to the computer **213** for storage in its storage **225** or a database associated with one or more servers. Under the direction of a program stored in its memory or accessible by the computer **213,** the computer **213,** which may be a laptop computer, desktop computer, workstation computer, electronic notepad, electronic tablet, smartphone or other device with at least one processor or other computer, processes the chemical shift spectrum in accordance with the teachings of the present invention to generate a report which may be output to a printer **226** or electronically stored and relayed to a desired email address or URL. Those skilled in this art will recognize that other output devices, such as a computer display screen, electronic notepad, smartphone and the like, may also be employed for the display of results.

It should be apparent to those skilled in the art that the functions performed by the computer **213** and its separate storage **225** may also be incorporated into the functions performed by the spectrometer's digital computer **211**. In such case, the printer **226** may be connected directly to the digital computer **211**. Other interfaces and output devices may also be employed, as are well-known to those skilled in this art.

Certain embodiments of the present invention are directed at providing methods, systems and/or computer program products that use BCAA evaluations that may be particularly useful in automated screening tests of clinical disease states and/or risk assessment evaluations for screening of *in vitro* biosamples.

Embodiments of the present invention may take the form of an entirely software embodiment or an embodiment combining software and hardware aspects, all generally referred to herein as a "circuit" or "module."

As will be appreciated by one of skill in the art, the present invention may be embodied as an apparatus, a method, data or signal processing system, or computer program product. Accordingly, the present invention may take the form of an entirely software embodiment, or an embodiment combining software and hardware aspects. Furthermore, certain embodiments of the present invention may take the form of a computer program product on a computer-usable storage medium having computer-usable program code means embodied in the medium. Any suitable computer readable medium may be utilized including hard disks, CD-ROMs, optical storage devices, or magnetic storage devices.

The computer-usable or computer-readable medium may be, but is not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a random access memory (RAM), a read-only memory (ROM), an erasable programmable read-only memory (EPROM or Flash memory), an optical fiber, and a portable compact disc read-only memory (CD-ROM). Note that the computer-usable or computer-readable medium could even be paper or another suitable medium, upon which the program is printed, as the program can be electronically captured, via, for instance, optical scanning of the paper or other medium, then compiled, interpreted or otherwise processed in a suitable manner if necessary, and then stored in a computer memory.

Computer program code for carrying out operations of the present invention may be written in an object oriented programming language such as Java7, Smalltalk, Python, Labview, C++, or VisualBasic. However, the computer program code for carrying out operations of the present invention may also be written in conventional procedural programming languages, such as the "C" programming language or even assembly language. The program code may execute entirely on the user's computer, partly on the user's computer, as a stand-alone software package, partly on the user's computer and partly on a remote computer or entirely on the remote computer. In the latter scenario, the remote computer may be connected to the user's computer through a local area network (LAN) or a wide area network (WAN), or the connection may be made to an external computer (for example, through the Internet using an Internet Service Provider).

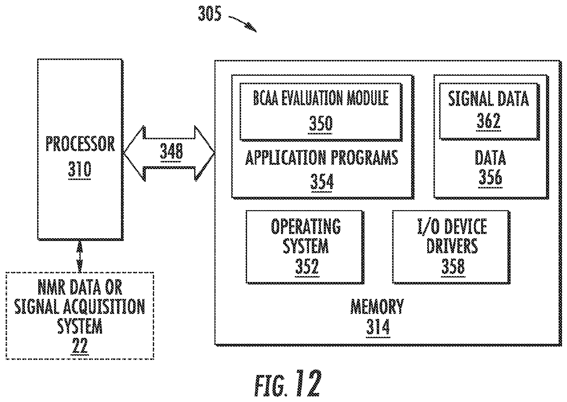

**Figure 12** is a block diagram of exemplary embodiments of data processing systems **305** that illustrates systems, methods, and computer program products in accordance with embodiments of the present invention. The processor **310** communicates with the memory **314** via an address/data bus **348**. The processor **310** can be any commercially available or custom microprocessor. The memory **314** is representative of the overall hierarchy of memory devices containing the software and data used to implement the functionality of the data processing system **305**. The memory **314** can include, but is not limited to, the following types of devices: cache, ROM, PROM, EPROM, EEPROM, flash memory, SRAM, and DRAM.

As shown in **Figure 12,** the memory **314** may include several categories of software and data used in the data processing system **305**: the operating system **352**; the application programs **354**; the input/output (I/O) device drivers **358;** a BCAA Evaluation Module **350;** and the data **356**. The BCAA Evaluation Module **350** can deconvolve NMR signal to reveal a defined NMR signal peak region in proton NMR spectra of a respective biosample to identify a level of BCAA. The system **305** may also include one or more of a Clinical Disease State Evaluation Module or a Risk Prediction Module that considers the level of the measured BCAA or generates a ratio, composite risk number, or multi-parameter risk model.

The data **356** may include signal (constituent and/or composite spectrum lineshape) data **362** which may be obtained from a data or signal acquisition system **320** (e.g., NMR spectrometer **22s** and/or analyzer **22**). As will be appreciated by those of skill in the art, the operating system **352** may be any operating system suitable for use with a data processing system, such as OS/2, AIX or OS/390 from International Business Machines Corporation, Armonk, NY, WindowsCE, WindowsNT, Windows95, Windows98, Windows2000 or WindowsXP from Microsoft Corporation, Redmond, WA, PalmOS from Palm, Inc., MacOS from Apple Computer, UNIX, FreeBSD, or Linux, proprietary operating systems or dedicated operating systems, for example, for embedded data processing systems.

The I/O device drivers **358** typically include software routines accessed through the operating system **352** by the application programs **354** to communicate with devices such as I/O data port(s), data storage **356** and certain memory **314** components and/or the image acquisition system **320.** The application programs **354** are illustrative of the programs that implement the various features of the data processing system **305** and can include at least one application, which supports operations according to embodiments of the present invention. Finally, the data **356** represents the static and dynamic data used by the application programs **354,** the operating system **352,** the I/O device drivers **358,** and other software programs that may reside in the memory **314.**

While the present invention is illustrated, for example, with reference to the Module **350** being an application program in **Figure 12,** as will be appreciated by those of skill in the art, other configurations may also be utilized while still benefiting from the teachings of the present invention. For example, the BCAA Module **350** may also be incorporated into the operating system **352,** the I/O device drivers **358** or other such logical division of the data processing system **305.** Thus, the present invention should not be construed as limited to the configuration of **Figure 12,** which is intended to encompass any configuration capable of carrying out the operations described herein.

In certain embodiments, the Module **350** includes computer program code for providing a level of BCAA which may be used as a marker to assess a clinical disease state or risk and/or to indicate whether therapy intervention is desired and/or track efficacy of a therapy or even an unintended consequence of a therapy.

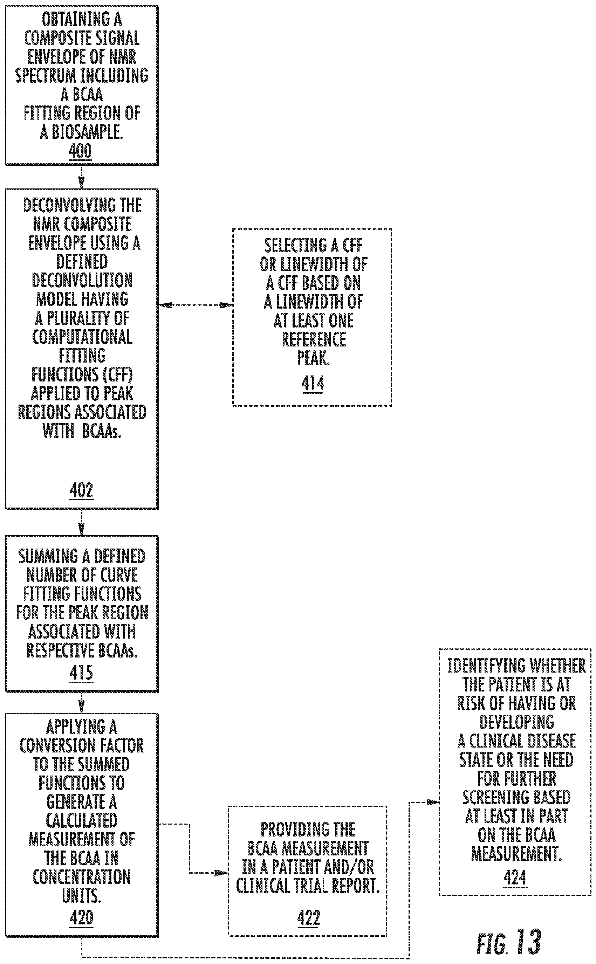

**Figure 13** is a flow chart of exemplary operations that can carry out embodiments of the present invention. A (measured) composite envelope NMR spectrum of NMR spectra of a fitting region of a biosample (e.g., blood plasma or serum) can be obtained (block **400**). The NMR composite signal envelope is electronically deconvolved using a defined model having at least one BCAA peak region centered at a defined chemical shift location (e.g., between about 0.85 ppm and about 1.10 ppm) (block **402**).

A defined number of (e.g., Lorentzian and/or Gaussian) curve fit functions for the peak region associated with a respective BCAA can be summed (block **415**). A conversion factor can be applied to the summed functions to generate a calculated measurement of BCAA (block **420**).

A CFF or linewidth of a CFF can be selected based on a linewidth of at least one reference peak that is associated with a measure of shim status of a spectrometer used to obtain the composite NMR spectrum (block **414**).

The BCAA measurement can be provided in a patient and/or clinical trial report (block **422**). The report can identify or alert as to whether he or she is at risk of having or developing a clinical disease state and/or whether additional screening may be appropriate, based at least in part on the BCAA measurement (block **424**).

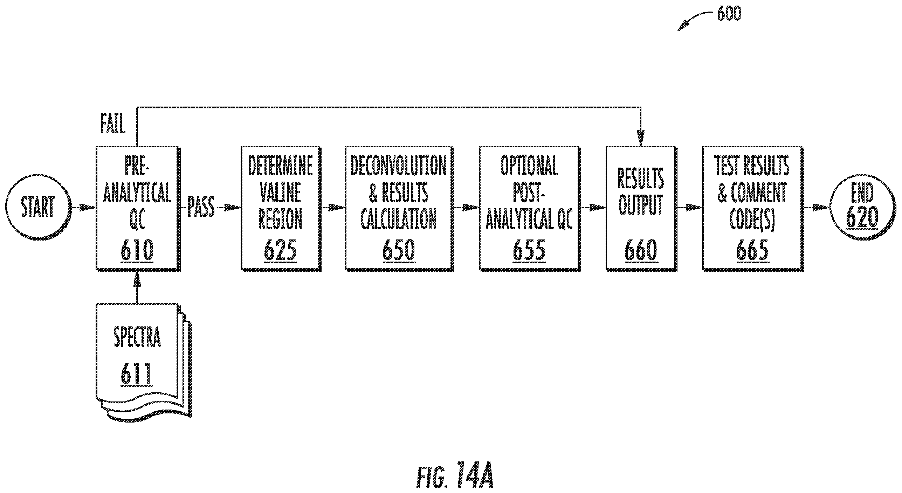

**Figures 14A-14C** are exemplary flow diagrams of operations that can be used to obtain NMR signal associated with valine according to embodiments of the present invention.

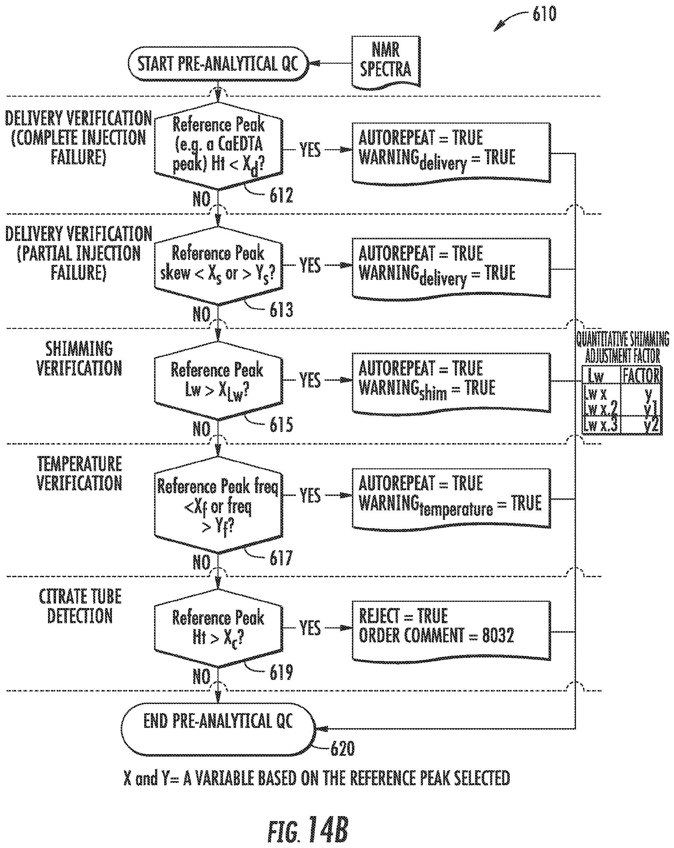

**Figure 14A** illustrates that a pre-analytical evaluation (block **610**) can occur before a BCAA region, such as, for example, a valine region, of the NMR signal is determined (block **625**), then deconvolved (block **650**). **Figure 14B** illustrates an exemplary pre-analytical evaluation **610** which includes delivery verification of the sample into the flow cell as either complete failure (block **612**) or partial injection failure (block **613**), shimming verification (block **615**), temperature verification (block **617**) and a citrate tube detection (failure) (block **619**), all using defined characteristics of signal associated with a defined diluent added to the sample. The shimming verification can include applying a quantitative shimming adjustment factor. Any suitable reference analyte may be used to generate the reference peak for use in the pre-analytical evaluation. The variables used in the evaluation will be determined based on the reference peak selected.

Referring again to **Figure 14A,** once the defined parameters are confirmed within limits, the pre-analytical quality control analysis can end (block **620**) and the determination of the valine region can be identified (block **625**) and the spectrum deconvolved and valine level calculated (block **650**). Optionally, a post-analytical quality control can be electronically performed (block **655**) and the results output (block **660**). The results can be included in a test report with comments, visual indicia of high or low and the like (block **665**).

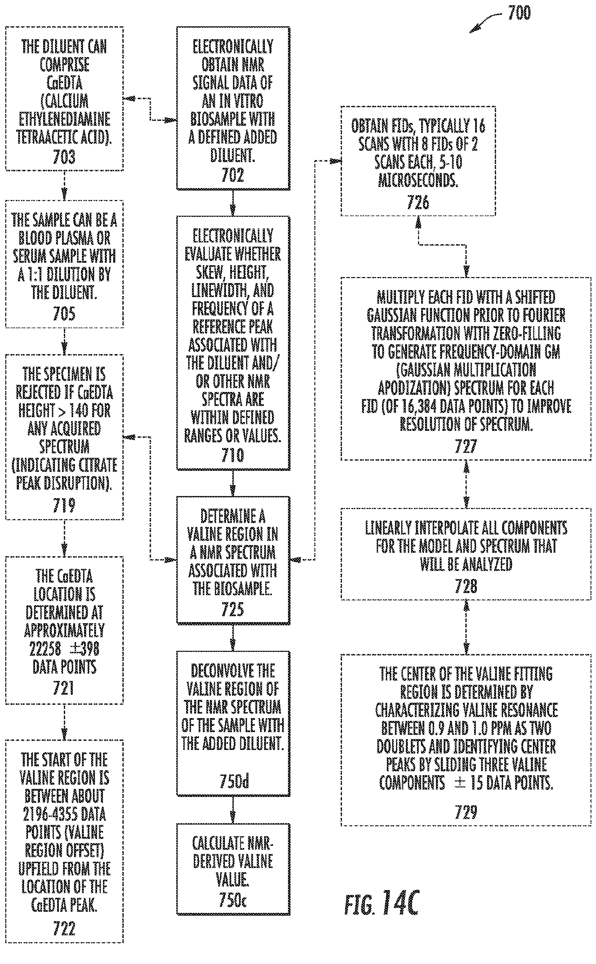

Referring to **Figure 14C,** an exemplary method/analysis **700** of operations that can be used to quantify valine are shown. NMR signal can be electronically obtained of an *in vitro* biosample with a defined added diluent (block **702**). The QC evaluation can be carried out (block **710**). The valine region is determined (block **725**). The valine region is deconvolved (block **750d**) and an NMR derived value of valine is calculated (**750c**).

The diluents can comprise a reference analyte, such as, for example, calcium ethylenediamine tetraacetic acid (Ca EDta) (block **703**) or other suitable diluent that creates a reliable peak and behaves in a predictable manner. Well established chemical shift or quantitation reference analytes include, for example, formate, trimethylsilylpropionate (and isotopically labeled isomers), and EDTA.

The pre-analytical quality control evaluation **710** can be based on inspection of characteristics of a reference peak, such as, for example, an CaEDTA reference peak, and the system or processor can be configured not to perform the Valine test unless the NMR spectra have been acquired under specified conditions such as those shown in **Figure 14B.** The sample temperature can be 47 ± 0.5 °C in the flow cell for NMR scans/signal acquisition. The sample can comprise diluents in a 1:1 ratio (block **705**) or other defined ratio (e.g., more sample, less diluents or more diluent; less sample, e.g., 2:1 or more sample, less diluents, e.g. 1:2).

The test sample can be rejected with a defined error code if CaEDTA height > 140 for any acquired spectrum (block **719**). This high value is indicative of detection of the citrate peak in conjunction with the CaEDTA peak. The citrate peak is introduced by collection of the specimen in an improper citrate tube. By disrupting the ability to locate the exact position of the CaEDTA peak, the citrate peak can disrupt the process for determining the Valine region.

The Valine region is located upfield relative to the position of the CaEDTA peak. The broad peaks beneath Valine are various methyl ($-CH_3-$) protons of lipoproteins. The CaEDTA location can be determined at approximately $22258 \pm 398$ data points (block **721**). The Valine region can be determined independently for each acquired spectrum. The Valine signal can be modeled with suitable data points using, for example, 25 data points (center $\pm$ 12 data points) for each peak of the quartet or 300 data points for the valine region of both doublets, but other numbers of data points may be used. The measurement of Valine can be carried out using one, two, three or all four peaks of the valine peak quartet.

All basis set spectra can be linearly interpolated before utilized by the non-negative least squares algorithm. The spectra to be analyzed and the basis set spectra can have a zero baseline offset modification before utilized by the non-negative least squares algorithm.

The start of the Valine region can be at about 2196-4355 data points, typically the latter when including both doublets, (the "Valine region offset") upfield from the location of the CaEDTA peak (block **722**). In some embodiments, the start of the valine region is at 4355 data points upfield from the location of the CaEDTA peak.

In some embodiments, the valine quantification is carried out by characterizing the valine resonances at between 0.0-1.01 ppm as two doublets. Three or more valine experimental spectra stepped by two data points can be used as basis sets to model valine signal. The center valine peaks can be located by sliding three valine components +/- 15 data points and determined through a least squares sum minimization. The valine signal can be modeled with a total of about 300 data points. The fitting algorithm may use the defined scalar coupling patterns of the experimental or computationally derived functions to help constrain and optimize the fitting.

Each basis set, including those used for the baseline but excluding the DC offset, are offset such that the lowest value is subtracted from the function (making the lowest point equal to 0). This prevents inclusion of a DC offset in the shapes they represent. The

Valine region from each acquired spectrum is deconvolved with a series of analyte and baseline functions which have been treated to the same type of pre-processing as the acquired spectra. The deconvolution coefficient for each component can be multiplied by an associated conversion factor. The resulting values are summed. Result values produced independently for each acquired spectrum can be averaged to generate final values to use in the measurement.

Data can be acquired using presaturation water suppression from a 1:1 diluted sample and can include about 16 scans, typically about 16 scans stored as 8 blocks of 2 (8 FIDs consisting of 2 scans each) (block **726**).

The pulse sequence used in conjunction with presaturation water suppression can optionally include a presaturation (water suppression) pulse and a suitable excitation pulse. For example, FIDs can be acquired with 9024 data points with a sweep width of 4496.4 Hz, but as known to those of skill in the art other data points and sweep widths may be used. Each FID can be multiplied with a shifted Gaussian function:

$$e^{-\left(\frac{(t-gfs)}{gf}\right)^2},$$

or in computer terms, exp(-((t-gfs)/gf)^2), where gfs = 0.2 seconds and gf = 0.2 seconds.

This can be performed prior to Fourier transformation with zero-filling which yields the frequency-domain GM spectrum for each FID consisting of 16,384 data points (block **727**). Each FID can be multiplied by a mathematical function to increase signal to noise (*e.g.,* a decaying exponential) and/or resolution (*e.g.,* a Gaussian type function and/or any shifted version thereof). The spectra can be phased using the calibration-specified phase value. The spectra can be scaled (multiplied) by a calibration-specified scaling factor. All basis set spectra can be linearly interpolated before utilized by the non-negative least squares algorithm. The spectra to be analyzed and the basis set spectra can have a zero baseline offset modification before utilized by the non-negative least squares algorithm (*e.g.*, all components used for the model and the spectrum that will be analyzed can be linearly interpolated) (block **728**). To determine the center of the valine fitting region, the valine resonances between 0.9 and 1.0 as two doublets can be characterized and the center peaks can be identified by sliding three valine components $\pm$ 15 data points (block **729**).

**Claims**

**1.** A method of determining concentrations of BCAAs, comprising:

placing at least one unfiltered *in vitro* serum or plasma sample in an NMR spectrometer having a static magnetic field $B_0$;

electronically obtaining an NMR spectrum of the sample that includes peaks associated with a plurality of BCAAs;

deconvolving the NMR spectrum using defined curve fitting functions, wherein the deconvolving is carried out using a baseline that is fit using protein and lipoprotein components;

computing unitless intensity signals from the deconvolved spectrum associated with respective BCAAs; then electronically calculating concentrations of the plurality of BCAAs using respective correlations of values of the computed intensity signals with known concentrations of corresponding BCAAs, wherein the BCAAs comprise valine, isoleucine and leucine.

2. The method of Claim 1, wherein the electronic calculation of concentrations:

includes applying a scalar concentration value defined as a linear function of concentrations versus unitless intensity signal correlations for each BCAA; or

is carried out for each of the plurality of BCAAs using a respective defined linear equation associated with concentrations over at least a normal biological range, optionally to provide a clinically relevant concentration measurement.

3. The method of Claim 1 or Claim 2, comprising, for each NMR spectrum of each respective sample before the deconvolving, electronically evaluating at least one reference peak in the NMR spectrum to assess magnetic field inhomogeneity and/or to select an appropriate fitting function or linewidth thereof, wherein linewidth and/or lineshape of the at least one reference peak is associated with magnetic field $B_0$ inhomogeneity and/or homogeneity.

4. The method of any preceding Claim, comprising applying scalar coupling information to BCAA peaks in the obtained NMR spectrum to thereby facilitate fitting during the deconvolving;

optionally wherein the scalar coupling information identifies multiple peaks which are separated by a consistent distance as measured in Hertz, irrespective of magnetic field strength, and wherein the deconvolving comprises fitting functions that can be constrained to a separation distance at or near the distance to thereby constrain the fitting.

5. The method of any preceding Claim, comprising, for each sample, before the deconvolving, electronically determining a location, lineshape and/or linewidth of at least one endogenous or exogenous reference peak(s) present in respective samples that has shape characteristics dependent on homogeneity of $B_0$.

6. The method of any preceding Claim, comprising, computationally determining lineshape and/or linewidth characteristics of each NMR spectrum for respective samples before the deconvolving step using at least one reference peak to account for differences in magnetic field homogeneity characteristics associated with a respective individual NMR spectrum, then selecting a defined deconvolution model with fitting functions having a linewidth and/or lineshape corresponding to the at least one reference peak such that different samples can be evaluated differently using (a) different fitting functions and/or (b) different linewidths.

7. The method of any preceding Claim, wherein valine and leucine are quantified using fewer scans associated with the obtained NMR spectrum than isoleucine.

8. The method of any preceding Claim, comprising, before the deconvolving, electronically evaluating at least one pH chemical-shift invariant reference peak in the obtained NMR spectrum associated with an endogenous or exogenous analyte or component of the sample, then determining a location of BCAA peaks in the NMR spectrum.

9. The method of any preceding Claim, wherein the electronically obtaining is carried out using:

between about 10 seconds to about 5 minutes of NMR acquisition time (AT) for a single NMR spectrum per sample; and optionally

between about 16-96 scans for a respective sample NMR spectrum, and wherein the electronically calculating the BCAA concentrations is carried out using a single NMR spectrum per sample.

10. The method of any preceding Claim, comprising transmitting a pulse sequence that attenuates signal from lipoproteins and proteins in the sample to a greater degree than signal from small molecule BCAAs, optionally wherein the pulse sequence comprises a Carr-Purcel-Meiboom-Gill (CPMG) pulse sequence with a defined delay that attenuates the lipoprotein and protein signals.

**11.** The method of Claim 10, wherein:

the CPMG pulse sequence has a delay of between about 60 ms to about 400 ms; or
the CPMG pulse sequence has a delay of about 100 ms; or
the CPMG pulse sequence has a delay of about 200 ms, or about 300ms, or about 400ms, and the method comprises electronically correcting the calculated concentrations for quantification errors associated with attenuation of signal intensity of the BCAAs associated with the CPMG pulse sequence.

**12.** The method of any preceding Claim, wherein the method includes transmitting a first lipoprotein pulse sequence, optionally a pulse sequence using a protocol with WET solvent suppression, including a series of solvent-directed selective pulses and pulsed field gradients followed by a read pulse and an acquisition time, and obtaining a first NMR spectrum associated with lipoproteins and proteins in the sample while the sample is in the NMR spectrometer and transmitting a second pulse sequence to obtain the NMR spectrum associated with the BCAAs that is configured to attenuate protein and lipoprotein signals before or after the first pulse sequence to obtain the NMR spectrum of the BCAAs, wherein both pulse sequences and subsequent NMR spectra are obtained within about 2 minutes to about 6 minutes of NMR signal acquisition time per sample.

**13.** The method of any preceding Claim, comprising flowing respective samples into the NMR spectrometer before the obtaining step, and wherein the obtaining is carried out by serially obtaining respective NMR spectra at a rate that is between about 50 and 250 unfiltered samples per 8 hours.

**14.** The method of Claim 7, wherein the NMR spectrum used to calculate valine and leucine is generated using between 16-96 scans, and wherein the NMR spectrum used to calculate isoleucine is generated using 96 scans.

**15.** The method of any preceding Claim, comprising electronically calculating a ratio or weighted index that is predictive of a clinical outcome or disease risk or identifies an efficacy of a drug or drug candidate:

wherein the ratio or weighted index has at least one of the following: (a) a numerator comprising at least one of the unitless signals or calculated concentrations of one or more of the BCAAs; (b) a denominator comprising at least one of the unitless signals or calculated concentrations of one or more of the BCAAs; or (c) a numerator and a denominator that comprises at least one of the unitless signals or calculated concentrations of one or more of the BCAAs; or
wherein the ratio or weighted index comprises in the denominator or numerator or in both the denominator and numerator at least one of the unitless signals or calculated concentrations of one or more of the BCAAs; or
wherein the ratio and/or weighted index comprises in the denominator or numerator or in both the denominator and numerator at least one of the unitless signals or calculated concentrations of one or more of the BCAAs and a unitless signal or concentration measurement of an amino acid.

**16.** A computer program product for evaluating *in vitro* patient serum or plasma biosamples, the computer program product comprising:
a non-transitory computer-readable storage medium having computer-readable program code embodied in the medium, the computer-readable program code comprising:

computer-readable program code configured to deconvolve a NMR spectrum with Branched Chain Amino Acid (BCAA) peaks of respective unfiltered patient samples using defined fitting functions, wherein the deconvolving is carried out using a baseline that is fit using protein and lipoprotein components;
computer-readable program code that computes unitless intensity signals from the deconvolved spectrum associated with respective BCAAs; and
computer-readable program code configured to provide concentrations of the BCAAs for the unitless intensity signals, using respective correlations of values of the computed intensity signals with known concentrations of corresponding BCAAs, wherein the BCAAs comprise valine, isoleucine and leucine.

**17.** A system, comprising:

an NMR spectrometer for acquiring at least one NMR spectrum of respective in vitro serum or plasma biosamples, the NMR spectrometer having a static magnetic field $B_0$; and
at least one processor in communication with the NMR spectrometer, the at least one processor configured, for a respective biosample, using the acquired at least one NMR spectrum:

deconvolve the NMR spectrum using defined fitting functions, wherein the deconvolving is carried out using a baseline that is fit using protein and lipoprotein components;

compute unitless intensity signals from the deconvolved spectrum associated with respective BCAAs; then calculate concentrations of the plurality of BCAAs using respective correlations of values of the computed intensity signals with known concentrations of corresponding BCAAs, wherein the BCAAs comprise valine, isoleucine and leucine.

**Patentansprüche**

1. Ein Verfahren zum Bestimmen von Konzentrationen von BCAAs, das Folgendes beinhaltet:

   Platzieren mindestens einer unfiltrierten In-vitro-Serum- oder -Plasmaprobe in ein NMR-Spektrometer, das ein statisches Magnetfeld $B_0$ aufweist;

   elektronisches Erhalten eines NMR-Spektrums der Probe, das Peaks umfasst, die mit einer Vielzahl von BCAAs assoziiert sind;

   Entfalten des NMR-Spektrums unter Verwendung von definierten Kurvenanpassungsfunktionen, wobei das Entfalten unter Verwendung einer Basislinie ausgeführt wird, die unter Verwendung von Protein- und Lipoproteinkomponenten angepasst wird;

   Errechnen einheitsloser Intensitätssignale von dem entfalteten Spektrum, die mit den BCAAs assoziiert sind; dann

   elektronisches Berechnen von Konzentrationen der Vielzahl von BCAAs unter Verwendung von jeweiligen Korrelationen von Werten der errechneten Intensitätssignale mit bekannten Konzentrationen entsprechender BCAAs, wobei die BCAAs Valin, Isoleucin und Leucin beinhalten.

2. Verfahren gemäß Anspruch 1, wobei die elektronische Berechnung von Konzentrationen:

   das Anwenden eines skalaren Konzentrationswerts umfasst, definiert als eine lineare Funktion von Konzentrationen gegenüber einheitslosen Intensitätssignalkorrelationen für jede BCAA; oder

   für jede von der Vielzahl von BCAAs unter Verwendung einer jeweiligen definierten linearen Gleichung ausgeführt wird, assoziiert mit Konzentrationen, die über mindestens einem normalen biologischen Bereich liegen, um optional eine klinisch relevante Konzentrationsmessung bereitzustellen.

3. Verfahren gemäß Anspruch 1 oder Anspruch 2, das Folgendes beinhaltet: für jedes NMR-Spektrum jeder jeweiligen Probe vor dem Entfalten, elektronisches Evaluieren von mindestens einem Referenz-Peak in dem NMR-Spektrum, um die Magnetfeld-Inhomogenität zu beurteilen und/oder eine angemessene Anpassungsfunktion oder Linienbreite davon zu wählen, wobei Linienbreite und/oder Linienform des mindestens einen Referenz-Peaks mit Magnetfeld-$B_0$-Inhomogenität und/oder -Homogenität assoziiert ist.

4. Verfahren gemäß einem der vorhergehenden Ansprüche, das das Anwenden von skalaren Kopplungsinformationen auf BCAA-Peaks in dem erhaltenen NMR-Spektrum beinhaltet, um somit das Anpassen während des Entfaltens zu erleichtern;

   wobei die skalaren Kopplungsinformationen optional mehrere Peaks identifizieren, die unabhängig von der Magnetfeldstärke durch einen gleichbleibenden Abstand, gemessen in Hertz, getrennt sind, und wobei das Entfalten das Anpassen von Funktionen beinhaltet, die auf einen Trennabstand an dem Abstand oder in der Nähe des Abstands beschränkt werden können, um somit das Anpassen zu beschränken.

5. Verfahren gemäß einem der vorhergehenden Ansprüche, das für jede Probe vor dem Entfalten das elektronische Bestimmen eines Orts, einer Linienform und/oder Linienbreite von mindestens einem endogenen oder exogenen, in jeweiligen Proben vorhandenen Referenz-Peak(s) beinhaltet, der Formcharakteristika aufweist, die von der Homogenität von Bo abhängig sind.

6. Verfahren gemäß einem der vorhergehenden Ansprüche, das Folgendes beinhaltet: rechnerisches Bestimmen von Linienform- und/oder Linienbreitencharakteristika jedes NMR-Spektrums für jeweilige Proben vor dem Entfaltungsschritt unter Verwendung von mindestens einem Referenz-Peak, um Unterschiede in Bezug auf Magnetfeld-Homogenitätscharakteristika zu berücksichtigen, die mit einem jeweiligen individuellen NMR-Spektrum assoziiert sind, dann Wählen eines definierten Entfaltungsmodells mit Anpassungsfunktionen, die eine Linienbreite und/oder Linienform aufweisen, die dem mindestens einen Referenz-Peak entspricht, sodass verschiedene Proben unter Verwendung von (a) verschiedenen Anpassungsfunktionen und/oder (b) verschiedenen Linienbreiten unterschiedlich evaluiert werden können.

7. Verfahren gemäß einem der vorhergehenden Ansprüche, wobei Valin und Leucin anhand von weniger mit dem erhaltenen NMR-Spektrum assoziierten Scans quantifiziert werden als Isoleucin.

8. Verfahren gemäß einem der vorhergehenden Ansprüche, das Folgendes beinhaltet: vor dem Entfalten, elektronisches Evaluieren von mindestens einem bei chemischer pH-Verschiebung invarianten Referenz-Peak in dem erhaltenen NMR-Spektrum, der mit einem endogenen oder exogenen Analyten oder einer Komponente der Probe assoziiert ist, dann Bestimmen eines Orts von BCAA-Peaks in dem NMR-Spektrum.

9. Verfahren gemäß einem der vorhergehenden Ansprüche, wobei das elektronische Erhalten unter Verwendung von Folgendem ausgeführt wird:

zwischen etwa 10 Sekunden bis etwa 5 Minuten langer NMR-Akquisitionszeit (AT) für ein einzelnes NMR-Spektrum pro Probe; und optional
zwischen etwa 16-96 Scans für ein jeweiliges Proben-NMR-Spektrum, und wobei das elektronische Berechnen der BCAA-Konzentrationen unter Verwendung eines einzelnen NMR-Spektrums pro Probe ausgeführt wird.

10. Verfahren gemäß einem der vorhergehenden Ansprüche, das das Übertragen einer Impulssequenz beinhaltet, die ein Signal von Lipoproteinen und Proteinen in der Probe stärker abschwächt als ein Signal von kleinmolekularen BCAAs, wobei die Impulssequenz optional eine Carr-Purcell-Meiboom-Gill(CPMG)-Impulssequenz mit einer definierten Verzögerung beinhaltet, die die Lipoprotein- und Proteinsignale abschwächt.

11. Verfahren gemäß Anspruch 10, wobei:

die CPMG-Impulssequenz eine Verzögerung von zwischen etwa 60 ms bis etwa 400 ms aufweist; oder
die CPMG-Impulssequenz eine Verzögerung von etwa 100 ms aufweist; oder
die CPMG-Impulssequenz eine Verzögerung von etwa 200 ms oder etwa 300 ms oder etwa 400 ms aufweist und das Verfahren das elektronische Berichtigen der berechneten Konzentrationen bezüglich Quantifizierungsfehlern, assoziiert mit der Abschwächung der Signalintensität der BCAAs, assoziiert mit der CPMG-Impulssequenz, beinhaltet.

12. Verfahren gemäß einem der vorhergehenden Ansprüche, wobei das Verfahren Folgendes umfasst: Übertragen einer ersten Lipoprotein-Impulssequenz, optional einer Impulssequenz unter Verwendung eines Protokolls mit WET-Lösungsmittelsuppression, einschließlich einer Reihe von lösungsmittelgerichteten selektiven Impulsen und Impulsfeldgradienten, gefolgt von einem Leseimpuls und einer Akquisitionszeit, und Erhalten eines ersten mit Lipoproteinen und Proteinen in der Probe assoziierten NMR-Spektrums, während sich die Probe in dem NMR-Spektrometer befindet, und Übertragen einer zweiten Impulssequenz, um das mit dem BCAAs assoziierte NMR-Spektrum zu erhalten, das dazu ausgelegt ist, Protein- und Lipoproteinsignale vor oder nach der ersten Impulssequenz abzuschwächen, um das NMR-Spektrum der BCAAs zu erhalten, wobei sowohl Impulssequenzen als auch nachfolgende NMR-Spektren innerhalb von etwa 2 Minuten bis etwa 6 Minuten der NMR-Signalakquisitionszeit pro Probe erhalten werden.

13. Verfahren gemäß einem der vorhergehenden Ansprüche, das das Strömen jeweiliger Proben in das NMR-Spektrometer vor dem Schritt des Erhaltens beinhaltet, und wobei das Erhalten durch serielles Erhalten jeweiliger NMR-Spektren bei einer Rate, die zwischen etwa 50 und 250 unfiltrierte Proben pro 8 Stunden liegt, ausgeführt wird.

14. Verfahren gemäß Anspruch 7, wobei das zum Berechnen von Valin und Leucin verwendete NMR-Spektrum anhand von zwischen 16-96 Scans erzeugt wird und wobei das zum Berechnen von Isoleucin verwendete NMR-Spektrum anhand von 96 Scans erzeugt wird.

15. Verfahren gemäß einem der vorhergehenden Ansprüche, das das elektronische Berechnen eines Quotienten oder gewichteten Indexes beinhaltet, der prädiktiv für ein klinisches Ergebnis oder Krankheitsrisiko ist oder eine Wirksamkeit eines Wirkstoffs oder Wirkstoffkandidaten identifiziert:

wobei der Quotient oder gewichtete Index mindestens eines der Folgenden aufweist: (a) einen Zähler, der mindestens eines der einheitslosen Signale oder berechneten Konzentrationen von einer oder mehreren der BCAAs beinhaltet; (b) einen Nenner, der mindestens eines der einheitslosen Signale oder berechneten Konzentrationen von einer oder mehreren der BCAAs beinhaltet; oder (c) einen Zähler und einen Nenner, der mindestens eines der einheitslosen Signale oder berechneten Konzentrationen von einer oder mehreren der BCAAs beinhaltet; oder

wobei der Quotient oder gewichtete Index im Nenner oder Zähler oder sowohl im Nenner als auch im Zähler mindestens eines der einheitslosen Signale oder berechneten Konzentrationen von einer oder mehreren der BCAAs beinhaltet; oder

wobei der Quotient und/oder gewichtete Index im Nenner oder Zähler oder sowohl im Nenner als auch im Zähler mindestens eines der einheitslosen Signale oder berechneten Konzentrationen von einer oder mehreren der BCAAs und ein einheitsloses Signal oder eine Konzentrationsmessung einer Aminosäure beinhaltet.

16. Ein Computerprogrammprodukt zum Evaluieren von biologischen In-vitro-Plasma- oder -Serumproben eines Patienten, wobei das Computerprogrammprodukt Folgendes beinhaltet:
ein nichtflüchtiges computerlesbares Speichermedium mit computerlesbarem Programmcode, der in dem Medium verkörpert ist, wobei der computerlesbare Programmcode Folgendes beinhaltet:

computerlesbaren Programmcode, der zum Entfalten eines NMR-Spektrums mit verzweigtkettigen Aminosäuren(BCAA)-Peaks von jeweiligen unfiltrierten Patientenproben unter Verwendung von definierten Anpassungsfunktionen ausgelegt ist, wobei das Entfalten unter Verwendung einer Basislinie ausgeführt wird, die unter Verwendung von Protein- und Lipoproteinkomponenten angepasst wird;
computerlesbaren Programmcode, der einheitslose Intensitätssignale von dem entfalteten Spektrum errechnet, die mit den jeweiligen BCAAs assoziiert sind; und
computerlesbaren Programmcode, der dazu ausgelegt ist, Konzentrationen der BCAAs für die einheitslosen Intensitätssignale unter Verwendung jeweiliger Korrelationen von Werten der errechneten Intensitätssignale mit bekannten Konzentrationen entsprechender BCAAs bereitzustellen, wobei die BCAAs Valin, Isoleucin und Leucin beinhalten.

17. Ein System, das Folgendes beinhaltet:

ein NMR-Spektrometer zum Akquirieren mindestens eines NMR-Spektrums jeweiliger biologischer In-vitro-Serum- oder -Plasmaproben, wobei das NMR-Spektrometer ein statisches Magnetfeld Bo aufweist; und mindestens einen Prozessor in Kommunikation mit dem NMR-Spektrometer, wobei der mindestens eine Prozessor für eine jeweilige biologische Probe unter Verwendung des akquirierten, mindestens einen NMR-Spektrums zu Folgendem ausgelegt ist:

Entfalten des NMR-Spektrums unter Verwendung definierter Anpassungsfunktionen, wobei das Entfalten unter Verwendung einer Basislinie ausgeführt wird, die unter Verwendung von Protein- und Lipoproteinkomponenten angepasst wird;
Errechnen einheitsloser Intensitätssignale von dem entfalteten Spektrum, die mit den jeweiligen BCAAs assoziiert sind; dann
Berechnen von Konzentrationen der Vielzahl von BCAAs unter Verwendung von jeweiligen Korrelationen von Werten der errechneten Intensitätssignale mit bekannten Konzentrationen entsprechender BCAAs, wobei die BCAAs Valin, Isoleucin und Leucin beinhalten.

**Revendications**

1. Procédé de détermination des concentrations de BCAA, comprenant :

le placement d'au moins un échantillon *in vitro* non filtré de sérum ou de plasma dans un spectromètre RMN ayant un champ magnétique statique Bo ;
l'obtention électronique d'un spectre RMN de l'échantillon qui comprend des pics associés à une pluralité de BCAA ;
la déconvolution du spectre RMN en utilisant des fonctions d'ajustement de courbe définies, dans lequel la déconvolution est réalisée en utilisant une valeur de référence qui est ajustée en utilisant des composants de protéines et de lipoprotéines ;
le calcul de signaux d'intensité sans unité du spectre déconvolutionné associé aux BCAA respectifs ; ensuite le calcul électronique des concentrations de la pluralité de BCAA en utilisant des corrélations respectives des valeurs des signaux d'intensité calculés avec des concentrations connues des BCAA correspondants, dans lequel les BCAA comprennent la valine, l'isoleucine et la leucine.

2. Procédé selon la revendication 1, dans lequel le calcul électronique des concentrations :

comprend l'application d'une valeur scalaire de concentration définie comme une fonction linéaire des concentrations par rapport à des corrélations de signaux d'intensité sans unité pour chaque BCAA ; ou est réalisé pour chacun de la pluralité de BCAA en utilisant une équation linéaire définie respective associée aux concentrations sur au moins une plage biologique normale, éventuellement pour fournir une mesure de concentration cliniquement pertinente.

3. Procédé selon la revendication 1 ou 2, comprenant, pour chaque spectre RMN de chaque échantillon respectif avant la déconvolution, l'évaluation électronique d'au moins un pic de référence dans le spectre RMN pour évaluer l'inhomogénéité du champ magnétique et/ou pour sélectionner une fonction d'ajustement appropriée ou une largeur de ligne de celle-ci, dans lequel la largeur de ligne et/ou la forme de ligne du au moins un pic de référence est associée à l'inhomogénéité et/ou l'homogénéité du champ magnétique Bo.

4. Procédé selon l'une quelconque des revendications précédentes, comprenant l'application d'informations de couplage scalaire aux pics de BCAA dans le spectre RMN obtenu pour faciliter ainsi l'ajustement pendant la déconvolution ;
éventuellement dans lequel les informations de couplage scalaire identifient de multiples pics qui sont séparés par une distance constante comme mesurée en Hertz, indépendamment de la force du champ magnétique, et dans lequel la déconvolution comprend des fonctions d'ajustement qui peuvent être contraintes à une distance de séparation à ou près de la distance pour ainsi contraindre l'ajustement.

5. Procédé selon l'une quelconque des revendications précédentes, comprenant, pour chaque échantillon, avant la déconvolution, la détermination électronique d'un emplacement, d'une forme de ligne et/ou d'une largeur de ligne d'au moins un des pics de référence endogènes ou exogènes présents dans les échantillons respectifs qui ont des caractéristiques de forme dépendantes de l'homogénéité de Bo.

6. Procédé selon l'une quelconque des revendications précédentes, comprenant la détermination de manière computationnelle des caractéristiques d'une forme de ligne et/ou d'une largeur de ligne de chaque spectre RMN pour des échantillons respectifs avant l'étape de déconvolution en utilisant au moins un pic de référence pour tenir compte des différences dans les caractéristiques d'homogénéité du champ magnétique associées à un spectre RMN individuel respectif, ensuite en sélectionnant un modèle de déconvolution défini avec des fonctions d'ajustement ayant une largeur de ligne et/ou une forme de ligne correspondant à au moins un pic de référence de sorte que différents échantillons peuvent être évalués différemment en utilisant (a) différentes fonctions d'ajustement et/ou (b) différentes largeurs de ligne.

7. Procédé selon l'une quelconque des revendications précédentes, dans lequel la valine et la leucine sont quantifiées en utilisant moins de balayages associés au spectre RMN obtenu que l'isoleucine.

8. Procédé selon l'une quelconque des revendications précédentes, comprenant, avant la déconvolution, une évaluation électronique d'au moins un pic de référence invariant au décalage chimique du pH dans le spectre RMN obtenu associé à un analyte ou composant endogène ou exogène de l'échantillon, ensuite en déterminant un emplacement des pics de BCAA dans le spectre RMN

9. Procédé selon l'une quelconque des revendications précédentes, dans lequel l'obtention électronique est réalisée en utilisant :

entre environ 10 secondes à environ 5 minutes du temps d'acquisition (TA) RMN pour un seul spectre RMN par échantillon ; et éventuellement
entre environ 16 à 96 balayages pour un spectre RMN d'échantillon respectif, et dans lequel le calcul électronique des concentrations de BCAA est réalisé en utilisant un seul spectre RMN par échantillon.

10. Procédé selon l'une quelconque des revendications précédentes, comprenant la transmission d'une séquence d'impulsions qui atténue un signal provenant des lipoprotéines et des protéines dans l'échantillon à un plus grand degré qu'un signal des BCAA à petites molécules, éventuellement dans lequel la séquence d'impulsions comprend une séquence d'impulsions Carr-Purcel-Meiboom-Gill (CPMG) avec un délai défini qui atténue les signaux des lipoprotéines et des protéines.

11. Procédé selon la revendication 10, dans lequel :

la séquence d'impulsions CPMG a un délai entre environ 60 ms à environ 400 ms ; ou
la séquence d'impulsions CPMG a un délai d'environ 100 ms ; ou
la séquence d'impulsions CPMG a un délai d'environ 200 ms, ou d'environ 300 ms, ou d'environ 400 ms, et le procédé comprend une correction électronique des concentrations calculées pour des erreurs de quantification associées à l'atténuation de l'intensité des signaux des BCAA associés à la séquence d'impulsions CPMG.

12. Procédé selon l'une quelconque des revendications précédentes, dans lequel le procédé comprend la transmission d'une première séquence d'impulsions de lipoprotéines, éventuellement une séquence d'impulsions utilisant un protocole avec une suppression de solvant WET, y compris une série d'impulsions sélectives dirigées par un solvant et des gradients de champ pulsés suivis par une impulsion de lecture et un temps d'acquisition, et l'obtention d'un premier spectre RMN associé à des lipoprotéines et à des protéines dans l'échantillon alors que l'échantillon est dans le spectromètre RMN et la transmission d'une seconde séquence d'impulsions pour obtenir le spectre RMN associé aux BCAA qui est configuré pour atténuer les signaux de protéines et de lipoprotéines avant ou après la première séquence d'impulsions pour obtenir le spectre RMN des BCAA, dans lequel les séquences d'impulsion et le spectre RMN sont obtenus en environ deux minutes à environ six minutes du temps d'acquisition du signal RMN par échantillon.

13. Procédé selon l'une quelconque des revendications précédentes, comprenant l'écoulement d'échantillons respectifs dans le spectromètre RMN avant l'étape d'obtention, et dans lequel l'obtention est réalisée en obtenant en série des spectres RMN respectifs à une vitesse qui se situe entre environ 50 et 250 échantillons non filtrés par huit heures.

14. Procédé selon la revendication 7, dans lequel le spectre RMN utilisé pour calculer la valine et la leucine est généré en utilisant entre 16 et 96 balayages, et dans lequel le spectre RMN utilisé pour calculer l'isoleucine est généré en utilisant 96 balayages.

15. Procédé selon l'une quelconque des revendications précédentes, comprenant un calcul électronique d'un rapport ou d'un indice pondéré qui est prédictif d'un résultat clinique ou d'un risque de maladie ou identifie l'efficacité d'un médicament ou d'un candidat médicamenteux :

dans lequel le taux ou l'indice pondéré a au moins un élément parmi les suivants : (a) un numérateur comprenant au moins un des signaux sans unité ou des concentrations calculées d'un ou de plusieurs des BCAA ; (b) un dénominateur comprenant au moins un parmi les signaux sans unité ou les concentrations calculées d'un ou de plusieurs des BCAA ; ou (c) un numérateur et un dénominateur qui comprennent au moins un des signaux sans unité ou des concentrations calculées d'un ou de plusieurs des BCAA ; ou
dans lequel le rapport ou l'indice pondéré comprend dans le dénominateur ou le numérateur ou dans le dénominateur et le numérateur au moins un parmi les signaux sans unité ou les concentrations calculées d'un ou de plusieurs des BCAA ; ou
dans lequel le rapport et/ou l'indice pondéré comprend dans le dénominateur ou le numérateur ou dans le dénominateur et le numérateur au moins un parmi les signaux sans unité ou les concentrations calculées d'un ou de plusieurs des BCAA et un signal sans unité ou une mesure de concentration d'un acide aminé.

16. Programme informatique pour l'évaluation *in vitro* d'échantillons biologiques d'un sérum ou d'un plasma d'un patient, le programme informatique comprenant :
un support de stockage non transitoire lisible par un ordinateur avec un code de programme lisible par un ordinateur intégré dans le support, le code de programme lisible par un ordinateur comprenant :

un code de programme lisible par un ordinateur configuré pour déconvolutionner un spectre RMN avec des pics d'acides aminés à chaîne ramifiée (BCAA) d'échantillons non filtrés respectifs d'un patient en utilisant des fonctions d'ajustement définies, dans lequel la déconvolution est réalisée en utilisant une valeur de référence qui est ajustée en utilisant des composants de protéines et de lipoprotéines ;
un code de programme lisible par un ordinateur qui calcule des signaux d'intensité sans unité du spectre déconvolutionné associé aux BCAA respectifs ; et
un code de programme lisible par un ordinateur configuré pour fournir des concentrations des BCAA pour les signaux d'intensité sans unité, en utilisant des corrélations respectives des valeurs des signaux d'intensité calculés avec des concentrations connues de BCAA correspondants, dans lequel les BCAA comprennent la valine, l'isoleucine et la leucine.

**17.** Système comprenant :

un spectromètre RMN pour acquérir au moins un spectre RMN d'échantillons biologiques in vitro respectifs de sérum ou de plasma, le spectromètre RMN ayant un champ magnétique statique Bo ; et
au moins un processeur en communication avec le spectromètre RMN, le au moins un processeur configuré, pour un échantillon biologique respectif, en utilisant le au moins un spectre RMN acquis, pour :

déconvoluer le spectre RMN en utilisant des fonctions d'ajustement définies, dans lequel la déconvolution est réalisée en utilisant une valeur de référence qui est ajustée en utilisant des composants de protéines et de lipoprotéines ;
calculer des signaux d'intensité sans unité du spectre déconvolutionné associé aux BCAA associés ; ensuite calculer des concentrations de la pluralité de BCAA en utilisant des corrélations respectives des valeurs des signaux d'intensité calculés avec des concentrations connues des BCAA correspondants, dans lequel les BCAA comprennent la valine, l'isoleucine et la leucine.

FIG. 1

Exemplary fitting of the BCAA region including valine, isoleucine and leucine. In this example these amino acids are modeled using experimentally derived components. The baseline is fit using experimentally derived protein and lipoprotein components. The chemical shift and coupling constant information for each of the BCAAs in this model are given below.

Chemical shifts and coupling constants in serum

| Val | 0.95 ppm, 7.14 (d) |
| | 1.00 ppm, 7.14 (d) |
| Ile | 0.90 ppm, 7.49 (t) |
| | 0.97 ppm, 7.11 (d) |
| Leu | 0.93 ppm, 6.22 (d) |
| | 0.92 ppm, 6.22 (d) |

FIG. 2

FIG. 3

D — Leucine: $y = 0.013x + 1.918$, $R^2 = 0.987$ (NMR deconvolution vs Spiked Leucine uM)

E — Valine: $y = 0.00112x + 0.230$, $R^2 = 0.987$ (NMR deconvolution vs Spiked Valine uM)

F — Isoleucine: $y = 0.0032x + 0.154$, $R^2 = 0.971$ (NMR deconvolution vs Spiked Isoleucine uM)

G — Leucine: $y = 0.987x + 145.55$, $R^2 = 0.987$ (NMR Leucine uM vs Spiked Leucine uM)

H — Valine: $y = 1.12x + 229.12$, $R^2 = 0.987$ (NMR Valine uM vs Spiked Valine uM)

I — Isoleucine: $y = 1.35x + 65.71$, $R^2 = 0.971$ (NMR Isoleucine uM vs Spiked Isoleucine uM)

Typical biological range (uM):
98.7 +/- 11.5

Typical biological range (uM):
212.3 +/- 62.3

Typical biological range (uM):
60.7 +/- 18.6

Reference biological ranges taken from Psychogios N, Hau DD, Peng J, Guo AC, Mandal R, et al. (2011) The Human Serum Metabolome. PLoS ONE 6(2): e16957. Table 3. Concentrations (mean +/- stdev) of serum metabolites as determined by NMR

FIG. 3

EP 2 906 956 B1

Valine

Isoleucine

Leucine

96 scan CPMG

16 scan CPMG

0.85 ppm

1.00 ppm

FIG. 4

30

FIG. 5

*FIG. 6*

*FIG. 7*

EP 2 906 956 B1

FIG. 8

*FIG. 9*

*FIG.* **10**

*FIG. 11*

305

PROCESSOR
310

348

NMR DATA OR
SIGNAL ACQUISITION
SYSTEM
22

BCAA EVALUATION MODULE
350

APPLICATION PROGRAMS
354

SIGNAL DATA
362

DATA
356

OPERATING
SYSTEM
352

I/O DEVICE
DRIVERS
358

MEMORY
314

*FIG. 12*

OBTAINING A
COMPOSITE SIGNAL
ENVELOPE OF NMR
SPECTRUM INCLUDING
A BCAA
FITTING REGION OF
A BIOSAMPLE.
400

DECONVOLVING THE
NMR COMPOSITE
ENVELOPE USING A
DEFINED
DECONVOLUTION
MODEL HAVING
A PLURALITY OF
COMPUTATIONAL
FITTING
FUNCTIONS (CFF)
APPLIED TO PEAK
REGIONS ASSOCIATED
WITH BCAAs.

402

SELECTING A CFF
OR LINEWIDTH OF
A CFF BASED ON
A LINEWIDTH OF
AT LEAST ONE
REFERENCE
PEAK.
414

SUMMING A DEFINED
NUMBER OF CURVE
FITTING FUNCTIONS
FOR THE PEAK REGION
ASSOCIATED WITH
RESPECTIVE BCAAs.
415

APPLYING A
CONVERSION FACTOR
TO THE SUMMED
FUNCTIONS TO
GENERATE A
CALCULATED
MEASUREMENT OF
THE BCAA IN
CONCENTRATION
UNITS.
420

PROVIDING THE
BCAA MEASUREMENT
IN A PATIENT AND/OR
CLINICAL TRIAL REPORT.
422

IDENTIFYING WHETHER
THE PATIENT IS AT
RISK OF HAVING OR
DEVELOPING
A CLINICAL DISEASE
STATE OR THE NEED
FOR FURTHER
SCREENING BASED
AT LEAST IN PART
ON THE BCAA
MEASUREMENT.
424

*FIG. 13*

FIG. 14A

600

START

SPECTRA 611

PRE-ANALYTICAL QC 610

FAIL

PASS

DETERMINE VALINE REGION 625

DECONVOLUTION & RESULTS CALCULATION 650

OPTIONAL POST-ANALYTICAL QC 655

RESULTS OUTPUT 660

TEST RESULTS & COMMENT (CODES) 665

END 620

EP 2 906 956 B1

FIG. 14B

X and Y= A VARIABLE BASED ON THE REFERENCE PEAK SELECTED

700

THE DILUENT CAN COMPRISE CaEDTA (CALCIUM ETHYLENEDIAMINE TETRAACETIC ACID).
703

ELECTRONICALLY OBTAIN NMR SIGNAL DATA OF AN IN VITRO BIOSAMPLE WITH A DEFINED ADDED DILUENT.
702

OBTAIN FIDs, TYPICALLY 16 SCANS WITH 8 FIDs OF 2 SCANS EACH, 5-10 MICROSECONDS.
726

THE SAMPLE CAN BE A BLOOD PLASMA OR SERUM SAMPLE WITH A 1:1 DILUTION BY THE DILUENT.
705

ELECTRONICALLY EVALUATE WHETHER SKEW, HEIGHT, LINEWIDTH, AND FREQUENCY OF A REFERENCE PEAK ASSOCIATED WITH THE DILUENT AND/OR OTHER NMR SPECTRA ARE WITHIN DEFINED RANGES OR VALUES.
710

MULTIPLY EACH FID WITH A SHIFTED GAUSSIAN FUNCTION PRIOR TO FOURIER TRANSFORMATION WITH ZERO-FILLING TO GENERATE FREQUENCY-DOMAIN GM (GAUSSIAN MULTIPLICATION APODIZATION) SPECTRUM FOR EACH FID (OF 16,384 DATA POINTS) TO IMPROVE RESOLUTION OF SPECTRUM.
727

THE SPECIMEN IS REJECTED IF CaEDTA HEIGHT > 140 FOR ANY ACQUIRED SPECTRUM (INDICATING CITRATE PEAK DISRUPTION).
719

DETERMINE A VALINE REGION IN A NMR SPECTRUM ASSOCIATED WITH THE BIOSAMPLE.
725

LINEARLY INTERPOLATE ALL COMPONENTS FOR THE MODEL AND SPECTRUM THAT WILL BE ANALYZED
728

THE CaEDTA LOCATION IS DETERMINED AT APPROXIMATELY 22258 ±398 DATA POINTS
721

DECONVOLVE THE VALINE REGION OF THE NMR SPECTRUM OF THE SAMPLE WITH THE ADDED DILUENT.
750d

THE CENTER OF THE VALINE FITTING REGION IS DETERMINED BY CHARACTERIZING VALINE RESONANCE BETWEEN 0.9 AND 1.0 PPM AS TWO DOUBLETS AND IDENTIFYING CENTER PEAKS BY SLIDING THREE VALINE COMPONENTS ±15 DATA POINTS.
729

THE START OF THE VALINE REGION IS BETWEEN ABOUT 2196-4355 DATA POINTS (VALINE REGION OFFSET) UPFIELD FROM THE LOCATION OF THE CaEDTA PEAK.
722

CALCULATE NMR-DERIVED VALINE VALUE.
750c

*FIG. 14C*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4933844 A
- US 6617167 B
- US 7243030 B
- US 8013602 B

**Non-patent literature cited in the description**

- **PSYCHOGIOS N. et al.** The Human Serum Metabolome. *PLOS ONE,* February 2011, vol. 6 (2
- **PSYCHOGIOS et al.** The Human Serum Metabolome. *PLoS ONE,* 2011, vol. 6 (2), e16957
- **LAWSON, CL ; HANSON RJ.** Solving Least Squares Problems, Englewood Cliffs. Prentice-Hall, 1974
- **OTVOS, JD ; JEYARAJAH, EJ ; BENNETT, DW.** *Clin Chem,* 1991, vol. 37, 377
- **BECKONERT, O. ; KEUN, H. C. ; EBBELS, T. M.** *Nat. Protoc,* 2007, vol. 2, 2996, , 2703
- **SIMPSON, A. J. ; BROWN, S. A.** *J. Magn. Reson.,* 2005, vol. 175, 340-346
- **SMALLCOMBE, S. H. ; PATT, S. L. ; KEIFER, P. A.** *J. Magn. Reson., Ser. A,* 1995, vol. 117, 295-303